(19) Europäisches Patentamt
European Patent Office
Office européen des brevets

(11) **EP 1 755 458 B1**

(12) **EUROPEAN PATENT SPECIFICATION**

(45) Date of publication and mention
of the grant of the patent:
**25.02.2015 Bulletin 2015/09**

(51) Int Cl.:
***A61B 8/00*** (2006.01)

(21) Application number: **05746359.8**

(86) International application number:
**PCT/US2005/015648**

(22) Date of filing: **05.05.2005**

(87) International publication number:
**WO 2005/107601 (17.11.2005 Gazette 2005/46)**

(54) **APPARATUS FOR THE SELECTIVE TREATMENT OF TISSUE**

GERÄT FÜR DIE SELEKTIVE BEHANDLUNG VON GEWEBE

APPAREIL POUR LE TRAITEMENT SELECTIF D'UN TISSU

(84) Designated Contracting States:
**AT BE BG CH CY CZ DE DK EE ES FI FR GB GR HU IE IS IT LI LT LU MC NL PL PT RO SE SI SK TR**

(30) Priority: **06.05.2004 US 568556 P**

(43) Date of publication of application:
**28.02.2007 Bulletin 2007/09**

(73) Proprietor: **Focus Surgery, Inc.
Indianapolis, In 46226 (US)**

(72) Inventors:
• **SEIP, Ralf
Indianapolis, IN 46256 (US)**
• **CHEN, Wo-Hsing
Fishers, IN 46038 (US)**
• **CARLSON, Roy, F.
New Palestine, IN 46163 (US)**
• **SANGHVI, Narendra, T.
Indianapolis, IN 46227 (US)**
• **DINES, Kris, A.
Indianapolis, IN 46202 (US)**
• **PENNA, Michael, A.
Indianapolis, IN 46226 (US)**
• **PFILE, Richard
Indianapolis, IN 46226 (US)**

(74) Representative: **Findlay, Alice Rosemary et al
Reddie & Grose LLP
16 Theobalds Road
London WC1X 8PL (GB)**

(56) References cited:
**WO-A-03/039370          WO-A2-01/57777
US-A- 5 722 411          US-A- 5 882 302**

**US-A- 5 993 389          US-B1- 6 618 620
US-B1- 6 618 620**

• **DUNCAN J S ET AL: "Medical image analysis using model-based optimization" PROCEEDINGS OF THE FIRST CONFERENCE ON VISUALIZATION IN BIOMEDICAL COMPUTING (CAT. NO.90TH0311-1) IEEE COMPUT. SOC. PRESS LOS ALAMITOS, CA, USA,, 22 May 1990 (1990-05-22), pages 370-377, XP010019036 ISBN: 978-0-8186-2039-3**
• **CHO P S ET AL: "Parametric Shape Modeling Using Deformable Superellipses for Prostate Segmentation" IEEE TRANSACTIONS ON MEDICAL IMAGING, IEEE SERVICE CENTER, PISCATAWAY, NJ, US, vol. 23, no. 3, 1 March 2004 (2004-03-01), pages 340-349, XP011108291 ISSN: 0278-0062**
• **SEIP R ET AL: "Automated HIFU treatment planning and execution based on 3D modeling of the prostate, urethra, and rectal wall", ULTRASONICS SYMPOSIUM, 2004 IEEE MONTREAL, CANADA 23-27 AUG. 2004, PISCATAWAY, NJ, USA,IEEE, vol. 3, 23 August 2004 (2004-08-23), pages 1781-1784, XP010784332, DOI: 10.1109/ULTSYM. 2004.1418172 ISBN: 978-0-7803-8412-5**
• **FEDEWA R J ET AL: "Automated treatment planning for prostate cancer HIFU therapy", ULTRASONICS SYMPOSIUM, 2005 IEEE ROTTERDAM, THE NETHERLANDS 18-21 SEPT. 2005, PISCATAWAY, NJ, USA,IEEE, vol. 2, 18 September 2005 (2005-09-18), pages 1135-1138, XP010899024, DOI: 10.1109/ULTSYM. 2005.1603050 ISBN: 978-0-7803-9382-0**

**Description**

**Notice**

This invention was made with government support under grant reference number 5R44DK059664-03 awarded by National Institutes of Health (NIH).

**Background of the Invention**

The present invention relates ultrasound systems and in particular to high intensity focused ultrasound ("HIFU") systems and the treatment of tissue with HIFU Systems. The treatment of tissue with high intensity focused ultrasound ("HIFU") energy is known in the art. For instance, HIFU may be used in the treatment of benign prostatic hyperplasia (BPH) and prostate cancer (PC). Further, it is known to use Doppler imaging to locate portions of tissue to be treated with HIFU energy.

HIFU Systems are known for the treatment of diseased tissue. An exemplary HIFU system is the Sonablate®-500 HIFU system available from Focus Surgery located at 3940 Pendleton Way, Indianapolis, Indiana 46226. The Sonablate® 500 HIFU system uses a dual-element, confocal ultrasound transducer which is moved by mechanical methods, such as motors, under the control of a controller. Typically one element of the transducer is used for imaging and the other element of the transducer is used for providing HIFU Therapy. A typical treatment procedure for treating the prostate with the Sonablate® 500 HIFU system includes using the imaging element of the transducer to create both two dimensional sector (or transverse) and two dimensional linear (or sagittal) ultrasound scans of the prostate capsule, manually defining treatment zones in multiple sector images (treatment sites placed in defined treatment zone by system), and using the therapy element of the transducer to provide HIFU Therapy to the patient. The treated site is then imaged to determine the effects of the HIFU Therapy. The positioning of the transducer, provision of HIFU Therapy, and post-imaging steps are repeated for each particular portion of tissue which is to be treated. All of these steps take place while the patient is immobilized on a treatment table.

The Sonablate® 500 HIFU system is particularly designed to provide HIFU Therapy to the prostate. However, as stated in U.S. Patent No. 5,762,066 the Sonablate® 500 HIFU system and/or its predecessors may be configured to treat additional types of tissue.

Further details of suitable HIFU systems may be found in US Patent No. 5,762,066; U.S. Abandoned Patent Application Serial No. 07/840,502 filed February 21, 1992, Australian Patent No. 5,732,801; Canadian Patent No. 1,332,441; Canadian Patent No. 2,250,081; U.S. Patent No. 5,036,855; U.S. Patent No. 5,117,832; U.S. Patent No. 5,492,126; U.S. Patent No. 6,685,640.

WO 01/57777 discloses a lesion library in which benign and malign lesions are stored for the use of a radiologist in identifying a lesion.

US 6618620 discloses a thermal treatment system including a heat applying element for generating thermal doses for ablating a target mass in a patient, a controller for controlling thermal dose properties of the heat applying element, an imager for providing preliminary images of the target mass and thermal images during the treatment, and a planner for automatically constructing a treatment plan, comprising a series of treatment sites that are each represented by a set of thermal dose properties. The planner automatically constructs the treatment plan based on input information including one or more of a volume of the target mass, a distance from a skin surface of the patient to the target mass, a set of default thermal dose prediction properties, a set of user specified thermal dose prediction properties, physical properties of the heat applying elements, and images provided by the imager.

**Summary of the invention**

As used herein the term "HIFU Therapy" is defined as the provision of high intensity focused ultrasound to a portion of tissue at or proximate to a focus of a transducer. It should be understood that the transducer may have multiple foci and that HIFU Therapy is not limited to a single focus transducer, a single transducer type, or a single ultrasound frequency. As used herein the term "HIFU System" is defined as a system that is at least capable of providing a HIFU Therapy.

An apparatus according to the invention is defined in claim 1.

There is also disclosed a method of providing treatment to a tissue treatment area including a plurality of tissue components comprising the steps of: generating ultrasound data related to the tissue treatment area; and automatically generating a proposed treatment plan of the tissue treatment area. The proposed treatment plan including a plurality of treatment sites selected to receive HIFU Therapy. The plurality of treatment sites being selected based on a three-dimensional model of a first tissue component located in the tissue treatment area. The three-dimensional model of the first tissue component being based on the generated ultrasound data In one example, the method further comprises the steps of: detecting blood flow in the tissue treatment area; and excluding a first portion of tissue from the proposed treatment plan, the exclusion of the first portion of tissue being based on the detection of blood flow at a location generally corresponding to the first portion of tissue. In one exemplary refinement, the exclusion of the first portion of tissue is further based on the location of the first portion relative to the three-dimensional model of the first tissue component. In another exemplary refinement, the tissue treatment area generally corresponds to a prostate of a patient, the first tissue component corresponds to a prostatic capsule, and the first portion generally corresponds to a neuro-vascular bundles and wherein the step of generating the ultrasound data includes the steps of positioning an ultrasound transducer proximate to the tissue treatment area by the transrectal insertion of the ultrasound transducer and obtaining multiple two-dimensional images of the tissue treatment area including a plurality of sector images and a plurality of linear images. In yet another example, the three-dimensional model is generated by the steps of: locating a first boundary trace of the first tissue component in a first set of ultrasound data generally corresponding to a first plane; locating a second boundary trace of the first tissue component in a second set of ultrasound data generally corresponding to a second plane, the second plane being generally orthogonal to the first plane; and computing a boundary surface of the first tissue component based on the first boundary trace and the second boundary trace. In still a further example, the method further comprises the steps of: presenting the proposed treatment plan on a display device along with a three dimensional representation of the tissue treatment area for review by a user; determining a first location within the tissue treatment area having blood flow associated therewith; further presenting on the display for review by a user an indicia to indicate the presence of blood flow at the first location, the indicia being positioned to correspond to the first location; receiving a modification to the proposed treatment plan from the user thereby generating a modified proposed treatment plan; and commencing the modified proposed treatment.

There is also disclosed a method of providing treatment to a tissue treatment area including a plurality of tissue components comprising the steps of: generating ultrasound data related to the tissue treatment area; generating blood flow data related to the tissue treatment area; determining the location of a first tissue component based on the ultrasound data; determining the location of a second tissue component based on the blood flow data; and automatically generating a proposed treatment plan of the tissue treatment area, the proposed treatment plan including a plurality of treatment sites, the plurality of treatment sites being selected such that HIFU Therapy is provided to the first tissue component and such that the second tissue component is excluded from HIFU Therapy. In one example, the blood flow data is generated by Doppler ultrasound imaging and wherein the location of the first tissue component is determined based on a three-dimensional model of the first tissue component, the three-dimensional model of the first tissue component being based on the ultrasound data. In one exemplary refinement, the location of the second tissue component is determined based on an indication of the presence of blood flow at the location of the second tissue component and the relative position of the of the location of the second tissue component and the three-dimensional model of the first tissue component. In still a further exemplary refinement, the method further comprises the steps of: presenting on a display device for review by a user; a three dimensional representation of the tissue treatment area; a plurality of treatment indicia, each of the treatment indicia corresponding to a respective treatment site, a representation of a boundary of the first tissue component, the boundary being determined from the three-dimensional model of the first tissue component; and a blood flow indicia indicating the location of the second tissue component, the blood flow indicia providing an indication of the amount of blood flow; receiving a modification to the proposed treatment plan from the user thereby generating a modified proposed treatment plan; and commencing the modified proposed treatment plan. In another example, the tissue treatment area generally corresponds to a prostate of a patient, the first tissue component generally corresponds to a prostatic capsule, and the second tissue component generally corresponds to a neuro-vascular bundles and wherein the step of generating the ultrasound data includes the steps of positioning an ultrasound transducer proximate to the tissue treatment area by the transrectal insertion of the transducer and obtaining multiple two-dimensional images of the tissue treatment area including a plurality of sector images and a plurality of linear images.

In one embodiment the plurality of treatment sites of the proposed treatment plan are selected to provide HIFU Therapy to a first tissue component, while excluding a second tissue component from HIFU Therapy, the location of the second tissue component being determined based on blood flow information obtained during the imaging mode of operation. In one example, the tissue treatment area generally corresponds to a prostate of a patient and wherein the transducer is contained within a probe, the probe being configured for transrectal insertion to position the transducer proximate to the tissue treatment area. In one exemplary refinement, the first tissue component generally corresponds to a prostatic capsule, and the second tissue component generally corresponds to a neuro-vascular bundles. In another exemplary refinement, the location of the first tissue component is determined based on a three-dimensional model of the first tissue component. The three-dimensional model being generated by the steps of: locating a first boundary trace of the first tissue component in a first image obtained during the imaging mode of operation, the first image generally corresponding to a first plane; locating a second boundary trace of the first tissue component in a second image obtained during the imaging mode of operation, the second image generally corresponding to a second plane, the second plane being generally orthogonal to the first plane; and computing a boundary surface of the first tissue component based on the first boundary trace and the second boundary trace. In still a further example, the apparatus further comprises a display device and the controller being configured to present with the display device for review by a user a three dimensional representation of the tissue treatment area, a plurality of treatment indicia, each of the treatment indicia corresponding to a respective treatment site in the proposed treatment plan, a representation of a boundary of the first tissue component, the boundary being determined from a three-dimensional model of the first tissue component; and a blood flow indicia indicating the location of the second tissue component. In one exemplary refinement, the apparatus further comprises an user input device and the controller being configured to generate a modified proposed treatment plan based on a requested modification received with the user input device.

In still a further exemplary embodiment the tissue treatment area generally corresponds to a prostate of a patient and wherein the transducer is contained within a probe, the probe being configured for transrectal insertion to position the transducer proximate to the tissue treatment area. In another example, the three-dimensional model of a first tissue component is generated by the steps of: locating a first boundary trace of the first tissue component in a first image obtained during the imaging mode of operation, the first image generally corresponding to a first plane; locating a second boundary trace of the first tissue component in a second image obtained during the imaging mode of operation, the second image generally corresponding to a second plane, the second plane being generally orthogonal to the first plane; and computing a boundary surface of the first tissue component based on the first boundary trace and the second boundary trace. In a further example, the apparatus further comprises a display device and the controller being configured to detect the presence of blood flow in the tissue treatment area of tissue and present with the display device for review by a user a three dimensional representation of the tissue treatment area including the three-dimensional model of the first tissue component, a plurality of treatment indicia, each of the treatment indicia corresponding to a respective treatment site in the proposed treatment plan, and a blood flow indicia indicating the location of blood flow in the tissue treatment area. In still a further example, the apparatus further comprises an user input device and the controller being configured generate a modified proposed treatment plan based on a requested modification received with the user input device.

There is further disclosed a method for treating tissue in a tissue treatment area including tissue components comprising the steps of: providing a HIFU system having software configured to provide therapy to diseased tissue by focusing ultrasound proximate to the diseased tissue and further configured to provide location information of blood flow in the tissue and location information on at least one of the tissue components; identifying potential treatment areas based on the location information of the tissue components; excluding potential treatment areas based on the location information of blood flow in the tissue treatment area; and providing therapy to all of the identified non-excluded treatment areas with the HIFU system In one example, the location information of the tissue components is presented to the a user by the following steps: identifying the location of blood flow with Doppler imaging; identifying the location of at least some of the tissue components with at least one of ultrasound 2-D imaging and ultrasound 3-D imaging; generating a three-dimensional model representation of the location of tissue components based on the location of blood flow and the ultrasound imaging information; and displaying the three-dimensional model on a display.

There is also disclosed a method of providing treatment to a tissue treatment area including a prostatic capsule and a neuro-vascular bundles comprising the steps of: imaging the tissue treatment area; and automatically generating a proposed treatment plan of the tissue treatment area, the proposed treatment plan including a plurality of treatment sites selected to receive HIFU Therapy, the plurality of treatment sites being selected to provide HIFU Therapy to the prostatic capsule and to exclude the neuro-vascular bundles from the provision of HIFU Therapy. In one example, the location of the neuro-vascular bundles is determined based on blood flow information obtained during the imaging of the tissue treatment area. In yet another example, the method further comprises the steps of: presenting the proposed treatment plan to a user for review; receiving a modification to the proposed treatment plan from the user; and generating a modified proposed treatment plan based on the received modification.

Additional features of the present invention will become apparent to those skilled in the art upon consideration of the following detailed description of the illustrative embodiment exemplifying the best mode of carrying out the invention as presently perceived.

## Brief Description of the Drawings

The detailed description of the drawings particularly refers to the accompanying figures in which:

Fig. 1 is a representative view of an exemplary HIFU System including a transducer which is to be positioned proximate to a tissue treatment area;

Fig. 2 is an isometric view of an exemplary embodiment of the HIFU System of Fig. 1 including an enlarged view of a probe tip, the probe tip being illustrated with various sector image planes that come to be imaged with a transducer at the probe;

Fig. 2A is a representation of a probe of the HIFU System of Fig. 2 illustrating two types of images which are to be obtained during an imaging of the tissue treatment area;

Fig. 3 illustrates an exemplary embodiment of the controller of Fig. 1;

Fig. 4A is a representative view of a first exemplary PW Doppler imaging system including multiple gates;

Fig. 4B is a representative view of a first exemplary CFI Doppler imaging system including multiple gates;

Fig. 4C is a representative view of a second exemplary CFI Doppler imaging system including multiple gates in software;

Fig. 5 illustrates an exemplary method of operation of the controller of Fig. 1;

Fig. 6A illustrates another exemplary method of operation of the controller of Fig. 1;

Fig. 6B is a representation of an automatic treatment planning module of Fig. 6A;

Fig. 7 illustrates an exemplary method of the automatic treatment planning module of Fig. 6B;

Fig. 8A illustrates an exemplary tracing/marking user interface to be shown with the display device of the system of Fig. 1 for indicating the location of various tissue components in the tissue treatment area;

Fig. 8B illustrates an exemplary screen shot of the display device of the system of Fig. 1 showing multiple sector and linear image views of the tissue treatment area to be potentially selected for tracing/marking the tissue components;

Fig. 9 illustrates exemplary shape models;

Fig. 10A illustrates an exemplary sector view for display with the display device of the system of Fig. 1, including indicia of proposed treatment sites of the proposed treatment plan and indicia or representations indicating sites or regions of blood flow;

Fig. 10B illustrates exemplary sector view for display with the display device of the system of Fig. 1, including indicia of proposed treatment sites of the proposed treatment plan;

Fig. 11 illustrates a plurality of representative sector views for display with the display device of the system of Fig. 1, each of the sector views to contain indicia of proposed treatment sites of the proposed treatment plan;

Fig. 12 illustrates a volume image of the tissue treatment area for display with the display device of the system of Fig. 1, the volume image containing indicia of proposed treatment sites of the proposed treatment plan;

Fig. 13 illustrates a volume image of the tissue treatment area for display with the display device of the system of Fig. 1, the volume image containing indicia of proposed treatment sites of the proposed treatment plan and shape models of the various tissue components, and indicia or representations indicating sites or regions of blood flow;

Fig. 14 is an exemplary representation of an exemplary lesion library; and

Fig. 15 illustrates several exemplary lesions of varying sizes from the lesion library of Fig. 14.

## Detailed Description of the Drawings

The present application is directed to the treatment of diseases of the prostate with a HIFU system. However, it should be understood that the HIFU system may be implemented to treat other diseased tissues located at other regions in the body.

As described more fully herein, in one embodiment, the apparatus of the present application generate an automatic treatment plan for treatment of a tissue treatment area containing the diseased tissue. The automatic treatment plan is tailored to treat the diseased tissue and to selectively exclude the treatment of portions of the tissue treatment area. In one embodiment, the portions of the tissue treatment area selected for exclusion in the treatment plan are selected based on the location of one or more tissue components, the detection of blood flow, and/or physician input. In the case of treating the prostate, the portions of the tissue treatment area selected for exclusion are selected to minimize side effects of the treatment such as impotency, erectile dysfunction and/or incontinence.

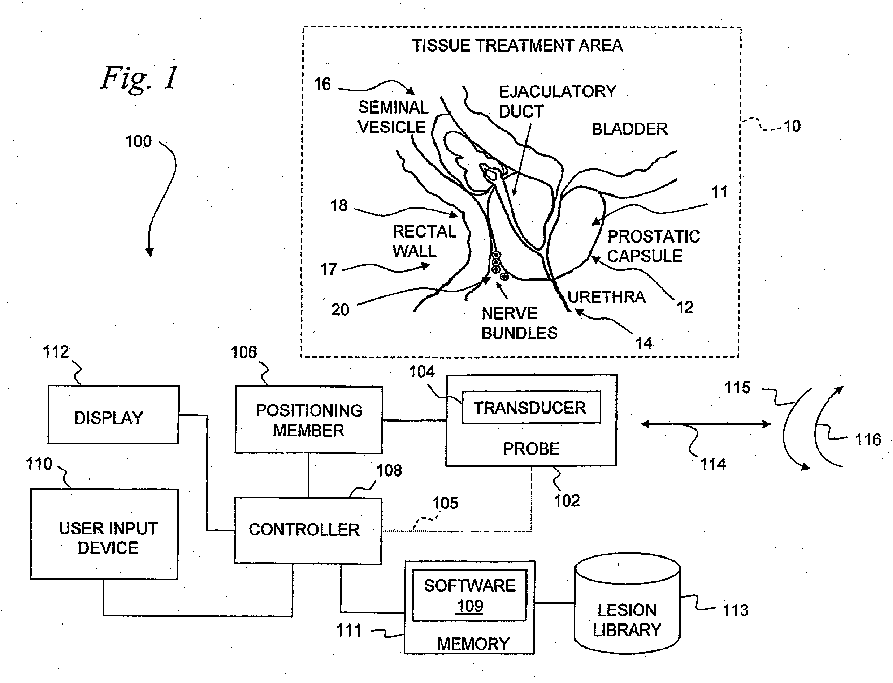

Referring to Fig. 1, several anatomical structures and their locations that are relevant to HIFU treatment planning for treatment of the prostate are shown. Referring to Fig. 1, a tissue treatment area or region 10 is shown. Tissue treatment area 10 is illustratively shown to include the following tissue components: a prostate 11 having a prostatic capsule 12, urethra 14, seminal vesicles 16, and a rectum 17 having a rectal wall 18. Further, neuro-vascular bundles ("NVB") 20 are shown wrapping tightly around the periphery of prostatic capsule 12. The NVB's are critical in the ability of the patient to achieve erections. As such, damage to NVB 20 may result in the patient experiencing impotence, erectile dysfunction and incontinence.

The location of the NVB 20 can be determined based on the locations of the vascular component of the NVB 20. The vascular component of the NVB 20, if healthy, includes blood flowing through respective blood vessels. Such blood flow may be detected with Doppler ultrasound imaging. Doppler ultrasound imaging techniques are well known in the art. Doppler ultrasound imaging may also be used to detect early stage prostate cancer in the patient due to the fact that early stage prostate cancer forms neo-vascularization.

An exemplary HIFU system 100 is shown in Fig. 1. HIFU system 100 includes a probe 102 having a transducer member 104, a positioning member 106, a controller 108 operably coupled to probe 102 and the positioning member 106, a user input device 110 (such as keyboard, trackball, mouse, and/or touch screen), and a display 112. Probe 102 is operably connected to controller 108 through positioning member 106. However, as indicated by line 105 probe 102 may be directly connected with controller 108. Positioning member 106 is configured to linearly position transducer member 104 along line 114 and to angularly position transducer member 104 in directions 115, 116.

In one exemplary embodiment, controller 108 includes software 109 located in memory 111. Software 109 controls the operation of HIFU System 100 including the imaging of the tissue treatment area 10, the automatic planning of a proposed HIFU treatment, and the provision of HIFU Therapy during treatment.

Transducer member 104 is positioned generally proximate to a region of tissue treatment area 10. In the case of the prostate, transducer 104 is positioned generally proximate to the prostate by the transrectal insertion of probe 102. Transducer member 104 is moved by positioning member 106 and controlled by controller 108 to provide imaging of at least a portion of the tissue in tissue treatment area 10 and to provide HIFU therapy to portions of the tissue within tissue treatment area 10. In one embodiment, prostatic capsule 12, urethra 14, seminal vesicles 16, rectal wall 18, are NVB 20 are included in the portions of tissue imaged. As such, HIFU system 100 may operate in an imaging mode wherein at least a portion of the tissue within tissue treatment area 10 may be imaged and in a therapy mode wherein HIFU therapy is provided to portions of the tissue within tissue treatment area 10.

In one embodiment, transducer member 104 is a single crystal two element transducer. A central element is used for imaging and a surrounding element is used for HIFU Therapy. In one embodiment, both elements work at 4 MHz. In another embodiment, the HIFU Therapy element operates at 4 MHz and the imaging element operates at 5-7 MHz. An exemplary transducer is disclosed in U.S. Patent No. 5,117,832.

However, one skilled in the art will appreciate that various transducer configurations may be implemented. In a one embodiment, transducer 104 is capable of providing imaging of at least a portion of the tissue within tissue treatment area 10 and of providing HIFU therapy to at least a portion of the tissue within tissue treatment area 10.

Various transducer geometries having a single focus or multiple foci and associated controls may be used including transducers which are phased arrays, such as the transducers disclosed in pending U.S. Patent Application Serial No. 11/070,371, filed on March 2,2005 ("'371 Application"). As explained in the '371 Application, at least one transducer disclosed therein has a scanning aperture. As such, the disclosed transducer does not require positioning member 106 to translate the disclosed transducer in direction 114 during imaging and treatment.

Additional exemplary transducers and associated controls are disclosed in U.S. Patent No. 5,762,066; U.S. Abandoned Patent Application Serial No. 07/840,502 filed February 21, 1992; Australian Patent No. 5,732,801; Canadian Patent No. 1,332,441; Canadian Patent No. 2,250,081; U.S. Patent No. 5,036,855; U.S. Patent No. 5,492,126; U.S. Patent No. 6,685,640. In one embodiment, a phased array transducer, Model No. 8EC4 available from Terason® located at 77-79 Terrace Hill Ave., Burlington, MA 01803 is incorporated into probe 102.

In a preferred embodiment, transducer 104 is capable of providing imaging information about the tissue in tissue treatment area 10 (such as a plurality of two-dimensional images), to provide HIFU therapy to at least a portion of the tissue in the tissue treatment area 10, and to provide Doppler imaging of at least a portion of the tissue in the tissue treatment area 10. In one embodiment, transducer 104 is a single transducer having at least two transducer elements. In another embodiment, transducer 104 is comprised of multiple transducers, each having one or more elements.

Probe 102 is configured to be positioned next to the rectal wall 18 of a patient and to be fixably secured relative to the patient during a treatment procedure as described below. By fixing the location of probe 102 relative to the patient it is possible to repeatably locate transducer member 104 relative to the patient with positioning member 106. Such repeatability is important due to requirements of the treatment procedure to first determine the location of various tissue components within the tissue treatment area 10 with ultrasound imaging, the determination of potential treatment zones or treatment sites based on the identified location information, and the subsequent placement of transducer member 104 to provide HIFU Therapy to locations in the tissue treatment area 10 corresponding to the treatment zones or treatment sites. Additional details of suitable ultrasound systems and methods of using high intensity focused ultrasound to treat tissue are disclosed in US Patent No. 5,762,066; U.S. Abandoned Patent Application Serial No. 07/840,502 filed February 21, 1992, Australian Patent No. 5,732,801; Canadian Patent No. 1,332,441; Canadian Patent No. 2,250,081; U.S. Patent No. 5,036,855; U.S. Patent No. 5,117,832; U.S. Patent No. 5,492,126; U.S. Patent No. 6,685,640.

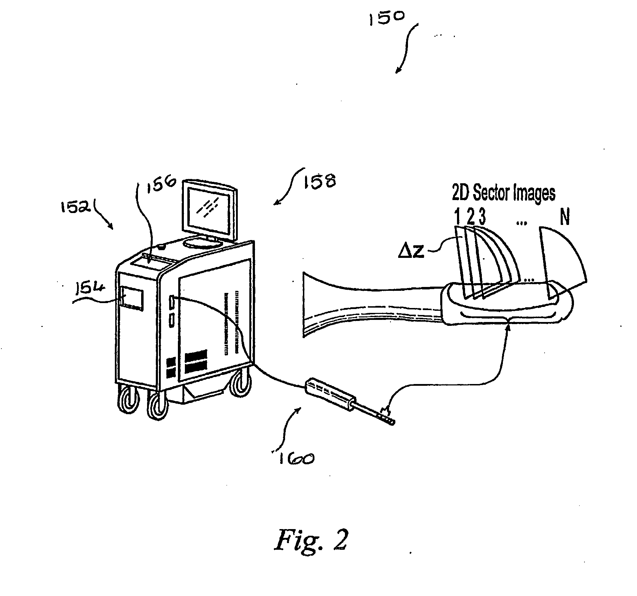

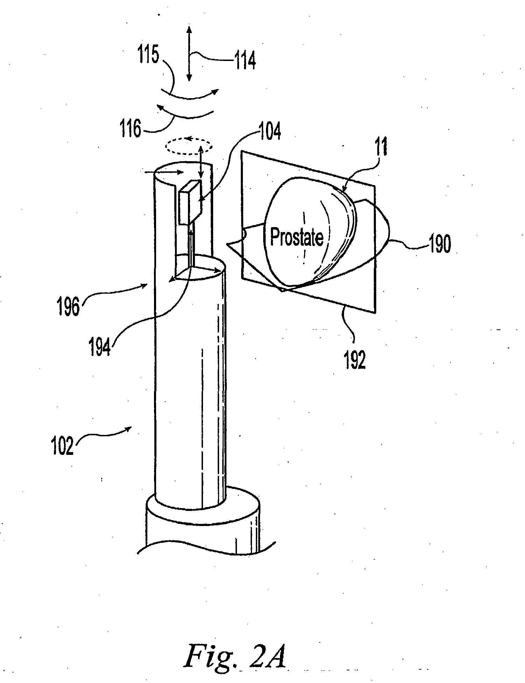

Referring to Fig. 2, an exemplary HIFU system 120 is shown, the Sonablate® 500 HIFU System available from Focus Surgery, Inc., located at 3940 Pendleton Way, Indianapolis, IN 46226. HIFU system 120 includes a console 122 which houses or supports a controller (not shown), such as a processor and associated software; a printer 124 which provides hard copy images of tissue 10 and/or reports; a user input device 126 such as a keyboard, trackball, and/or mouse; and a display 128 for displaying images of tissue 10 and software options to a user, such as a color display. Further shown is a probe 130 which includes a transducer member (not shown), and a positioning member (not shown). The Sonablate® 500 HIFU System further includes an articulated probe arm (not shown) which is attached to the operating room bed or surgical table (not shown). The articulated probe arm orients and supports probe 130. The Sonablate® 500 HIFU System further includes a chiller (not shown) which provides a water bath for the transducer member of probe 130 to remove heat from the transducer member during the provision of HIFU Therapy. In one embodiment, the software and/or hardware of the Sonablate® 500 HIFU System is modified to incorporate the functioning of the present invention.

Ultrasound system 100 is configured to use Doppler imaging techniques to identify the location of rapidly moving bodies in the tissue treatment area 10, such as blood flow associated with NVB 20. As explained below, such location information related to the location of blood flow is used in determining the location of various tissue components, such as NVB 20 so that these zones may be excluded from the treatment plan. In one embodiment described herein, the treatment plan is based on an automatically generated proposed treatment plan which takes into account the location of the NVB to exclude the NVB from the treatment plan.

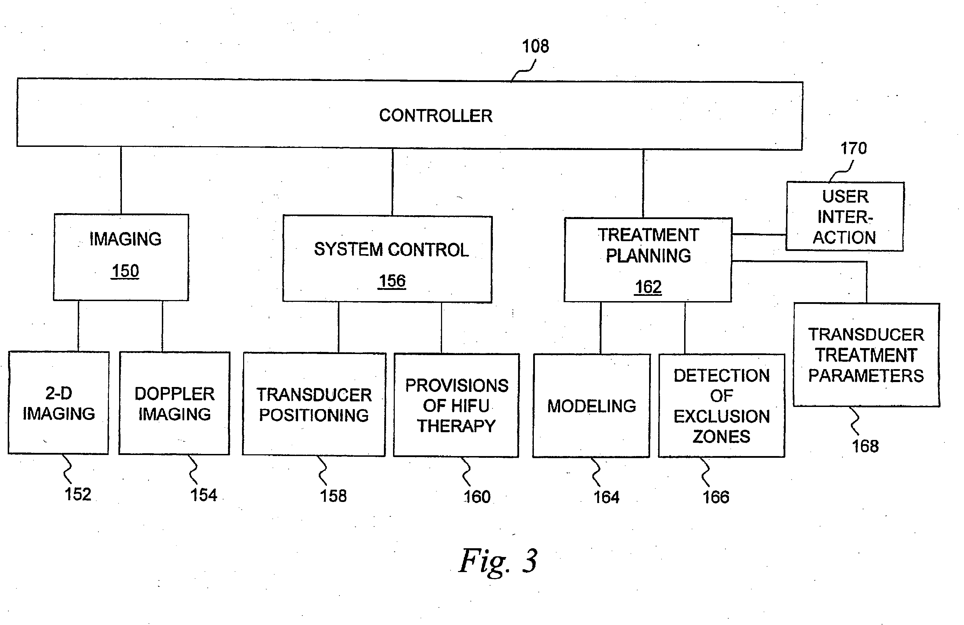

Referring to Fig. 3, an exemplary embodiment of controller 108 is illustrated. Controller 108 includes an imaging module 150 which controls the imaging of the tissue treatment area 10, a system control module 156 which controls various aspects of the system such as transducer positioning 158 and the provision of HIFU Therapy 160, and a treatment planning module 162 which develops a proposed treatment plan for treating the tissue in the tissue treatment area 10. Imaging module 150, as discussed herein, includes the imaging of the tissue treatment area by a plurality of two-dimensional images such as sector and linear images, as represented by 2-D imaging 152. Imaging module 150, as discussed herein, further includes the use of Doppler Imaging to determine the location(s) of blood flow in the tissue treatment area, as represented by Doppler Imaging 154. Treatment planning module 162, as discussed herein, generates a proposed treatment plan for treating the tissue in the tissue treatment area 10. Treatment planning module 162 includes a modeling component 164 which models tissue components in the tissue treatment area 10, a detection of exclusion zones component 166 which determines the location of exclusion zone as discussed herein, a transducer/treatment parameters component 168 which provides input on the system characteristics, and a user interaction component 170 which provides information about a proposed treatment plan to a user and receives modifications from a user.

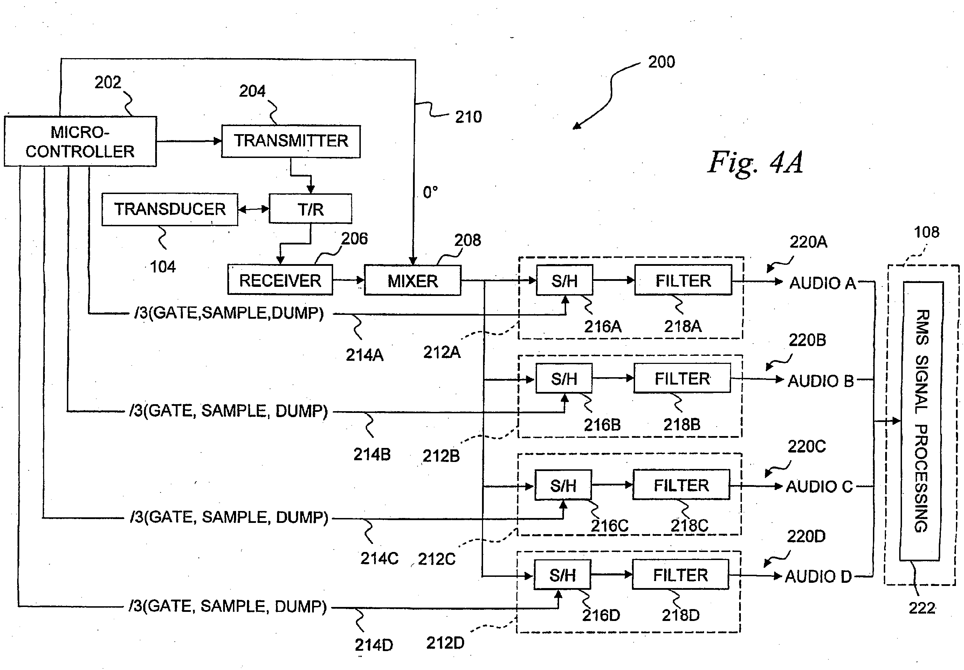

An exemplary embodiment of the Doppler System 154 of controller 108 is discussed below in connection with Figs. 4A-C. As explained herein, the Doppler system may be implemented as software and as a combination of software and hardware. Further, a Pulsed-Wave (PW) Doppler system and a Color Flow Imaging (CFI) Doppler system are disclosed.

Referring to Fig. 4A, a first PW Doppler system 200 is shown. System 200 includes a digital micro-controller 202 which provides a signal to an analog transmitter 204 instructing the transmitter to transmit an ultrasound signal with transducer 104. In a preferred embodiment, transducer 104 uses a single element for Doppler imaging. In one embodiment, the transmitted signal is about 4 microseconds (μsec) in duration and at a frequency of about 4 MHz. Echo signals (reflected from the tissue treatment area) are received by transducer 104 subsequent to the transmitted signal and are provided to an analog receiver 206. Receiver 206 passes the received echo signals onto a mixer 208 which mixes the analog echo signal with a reference signal (indicated by line 210) from micro-controller 202. The reference signal has the same frequency as the transmitted signal and is used to remove this high frequency carrier signal, from the received echo signal; thereby leaving the low frequency Doppler signal. In one example, the low frequency Doppler signal is between about 400 Hz to about 2 kHz.

The resultant Doppler signal is then provided to a series of gates 212a, 212b, 212c, 212d. The particular gate 212 that receives the resultant Doppler signal is controlled by micro-controller 202 as represented by lines 214a, 214b, 214c, 214d. In each gate (when the respective gate is active), the Doppler signal is sampled and held, as illustrated by block 216a, 216b, 216c, 216d. Further, the resultant sampled Doppler signal is filtered with a bandpass filter 218a, 218b, 218c, 218d. In one example, the bandpass filter is configured to pass frequencies in the range of about 400 Hz to about 2 kHz.

Each gate 212 is activated by microcontroller 202 for a specified period of time. For example, for an echo signal whose useful duration (the useful duration in one embodiment being the time frame generally equal to expected depth of the tissue being imaged) is n μsec, gate 212a is activated from 0 to n/4 μsec, gate 212b is activated from n/4 μsec to n/2 μsec, gate 212c is activated from n/2 μsec to 3n/4 μsec, and gate 212d is active from 3n/4 μsec to n μsec. If only a single gate 212 was used, then four separate transmitted and received echo pairs would need to be used to cover the same depth of tissue as the four gate system 200 illustrated. As such, by using multiple gates 212a, 212b, 212c, 212d more of the echo signal received by transducer 104 may be processed for the presence of Doppler information as a function of depth at the same time and hence more depth of the tissue treatment area 10 may be reviewed with a given transmitted signal and received echo pair. This reduces the amount of time needed to obtain Doppler information about the tissue treatment area 10.

In one embodiment, microcontroller 202, transmitter 204, receiver 206, mixer 208, sampling/hold 216 and filter 218 are all included on a circuit board such as an audio board. Each gate 212 contained on the circuit board having an output 220. This output 220 provides the filtered signal to one of a speaker (not shown) for auditory detection of the presence of blood flow and controller 108 for further processing. In one embodiment, controller 108 processes the output signal by root mean square (RMS) techniques, as represented by block 222, for the presence of blood flow (as indicated by the shift in frequency due to the Doppler effect). RMS processing 222 for the presence of blood flow is well known in the art and may be carried out by hardware processing and/or software processing. In an alternative embodiment, Fast Fourier Transform (FFT) spectrum integration (or power spectrum) may be used to detect the Doppler phase change. FFT typically has a higher Signal to Noise Ratio (SNR) than RMS processing.

In the above exemplary system, the microcontroller 202 manipulates the frequency and repetition rate of the Doppler transmit pulse, controls the width and depth of the multiple receiving gates 212 for analog receiving processing, and generates the reference signal 210 for demodulating the Doppler echo in analog transmit/receive circuit section. In an alternative embodiment, the multiple gates 212 of system 200 shown in Fig. 4A are replaced by software processing wherein the received echo signal is digitized and stored in memory 111 associated with controller 108. Software 109 then processes the data to separate the time signal by different gate locations. This approach requires a 16 bit or higher resolution analog to digital converter and more memory and processing capability than the above illustrated system 200.

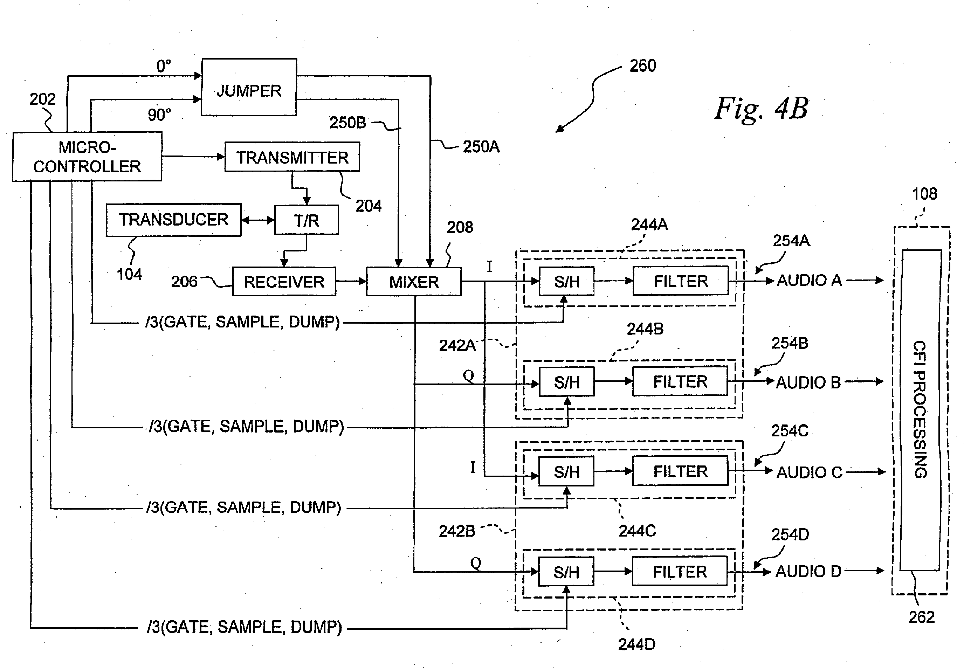

System 200 described in connection with Fig. 4A is able to detect the presence of blood flow. However, system 200 is not able to distinguish the direction of blood flow. As illustrated in Fig. 4B, system 200 may be modified to produce system 260 which is capable of determining the direction of blood flow. System 260 unlike system 200 has two gates 242a and 242b, each having two channels 244a, 244b and 244c, 244d. However, more gates 242 may be added to system 260 such that it has an equal number of gates 242 as system 200.

System 260 has two reference signals 250a and 250b that are provided to mixer 208. Reference signals 250a, 250b are similar to reference signal 210 in that they are at the carrier frequency (in one example about 4 MHz), but reference signal 250b is 90° out of phase from reference signal 250a. Each gate 242 of system 240 samples the mixed received echo signal and 0° reference signal on a first channel (I channel) 244a and 244c, respectively, and the mixed received echo signal and 90° reference signal on a second channel (Q channel) 244b and 244d, respectively. The combination of these two channels are then processed by well known color flow imaging techniques indicated by color flow imaging routine (CFI) 262. CFI requires two channels 244 for each gate 242, both the I and Q channels. Further, multiple successive transmitted signals and their respective echo signals are required to estimate mean velocity of the flow rate of the blood. An exemplary algorithm for the estimation of the mean velocity of the flow rate of the blood is provided below:

$$\text{Mean velocity } \varpi = \frac{1}{T}\tan^{-1}\left\{ \frac{\sum_{i=1}^{n} Q(i)I(i-1) - I(i)Q(i-1)}{\sum_{i=1}^{n} I(i)I(i-1) + Q(i)Q(i-1)} \right\} \qquad (1)$$

wherein

n = number of transmitted/echo signal pairs,
T = pulse repetition interval,
I = the signal from the I channel, and
Q = the signal from the Q channel.

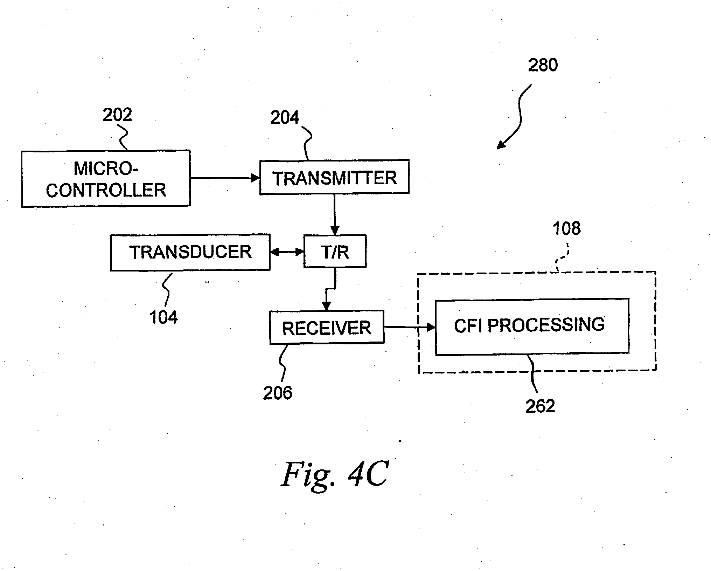

Referring to Fig. 4C, the multiple gate system 260 may be carried out in software, system 280, wherein the CFI Processing 262 further includes a cross-correlation function to detect the time shift of gated echoes in successive RF signal. The hardware does not require mixer 208, sample/hold circuit 216, and filters 218. All data processing is in the digital domain. However, a low signal-to-noise ration processed in the digital domain with digitization errors may affect the results of flow velocity estimation.

The location information obtained by HIFU System 100, sector and linear images and Doppler information, is used to provide on display 112 a representation of the tissue treatment area 10. In one embodiment, the representation of the tissue treatment area 10 includes one or more two-dimensional views of the tissue treatment area, such as one or more sector views and one or more linear views. In one example, traditional 2-D ultrasound imaging is used to generate sectors views, such as a sector view in sector plane 190 in Fig. 2A and linear views, such as a linear view in linear plane 192 in Fig. 2A. In one embodiment as discussed herein, the displayed view, preferably a sector view, provides a representation or icon to indicate the location of blood flow as determined by using Doppler imaging techniques. In another example, multiple sector views 200 are arranged on display 112 so that the physician can see multiple sectors of the tissue treatment area 10. In still another example, both sector views and linear views are arranged on display 112 so that the physician may see multiple sectors of the tissue treatment area 10. In all of the above examples, an automatically generated proposed treatment plan, or at least a portion of the proposed treatment plan may be displayed as well.

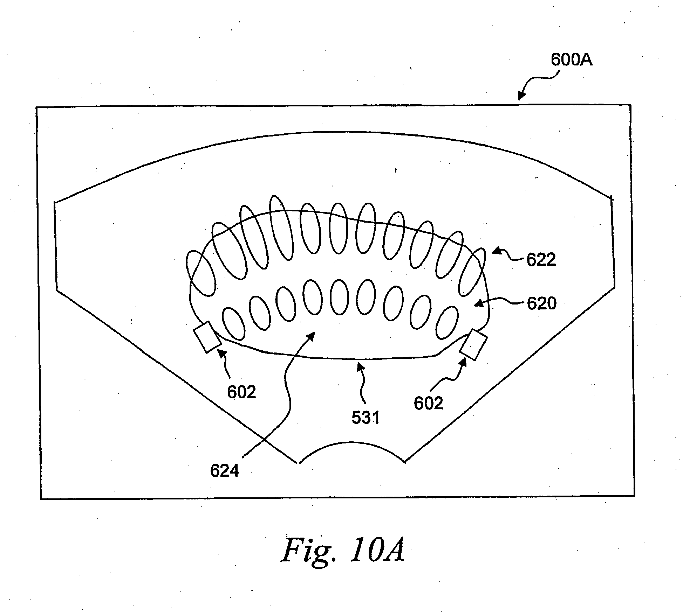

In one embodiment the icon or representation of the location of blood flow is shown as an abstracted representation, such as box 602 in Fig. 10A. This provides a general indication of the location of blood flow. The icon or representation 602 may be colored to offset itself from the background. In another embodiment, the icon or representation of the location of blood flow is one or more color pixels. The color pixels are typically colored to offset themselves from the background, such as red or blue. Further, in one example, only the pixels (either a two-dimensional pixel for a sector view or a three-dimensional pixel for a volume view) which corresponds to locations indicated as having blood flow are colored. This provides more exact location information as well as shape information of the blood flow region. In one embodiment, the brightness, color, or other indicia of the representation is an indication of the amount or velocity of blood flow or the direction of the blood flow. For instance, the representation or icon may be brighter to indicate higher volumes or velocities of blood flow.

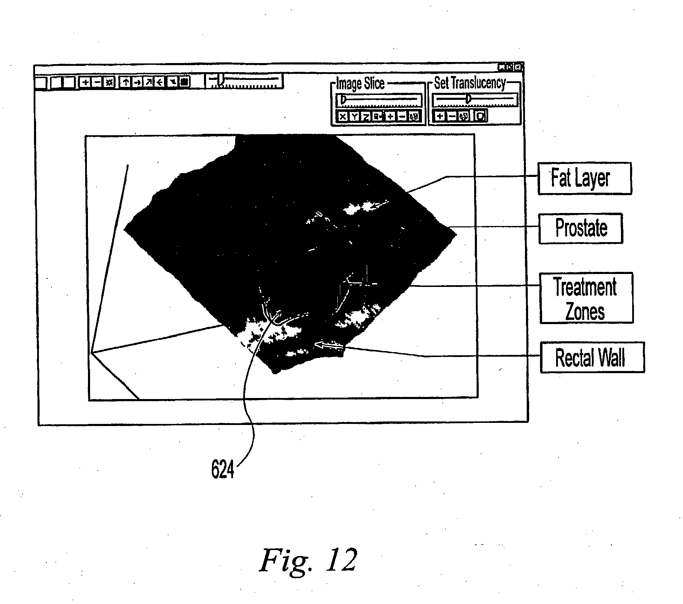

In addition to the above discussed 2-D imaging capability, in one embodiment, HIFU System 100 is configured to provide three-dimensional imaging of the tissue treatment area 10. In one embodiment, HIFU System 100 displays a volume image of the tissue treatment area 10, the volume image being generated from multiple 2-D images (see Fig. 12 for example). In a preferred embodiment, HIFU System 100 generates a three-dimensional model representation of the location of the tissue components of tissue treatment area 10. Details of a preferred method to generate three-dimensional model of tissue components in the tissue treatment area 10 is provided herein.

As described in more detail herein, various techniques are used to model the location of tissue components expected to be located in the tissue treatment area 10. In the instance wherein the tissue treatment area 10 corresponds to the area surrounding prostate 11, expected tissue components include rectal wall 18, urethra 14, prostatic capsule 12, seminal vesicles 16, and/or neuro-vascular bundles ("NVB") 20. In one embodiment, the three-dimensional representation of one or more tissue components may be manipulated through input from user input device 110 to change the orientation of the respective three-dimensional representation. In this way a physician may virtually review all sides of the representations of the tissue components of the tissue treatment area 10.

The location information determined by the Doppler imaging, 2-D imaging and/or the 3-D imaging with HIFU System 100 in a preferred embodiment is utilized to determine an appropriate treatment procedure for treating at least a portion of the tissue in the tissue treatment area 10 with HIFU Therapy.

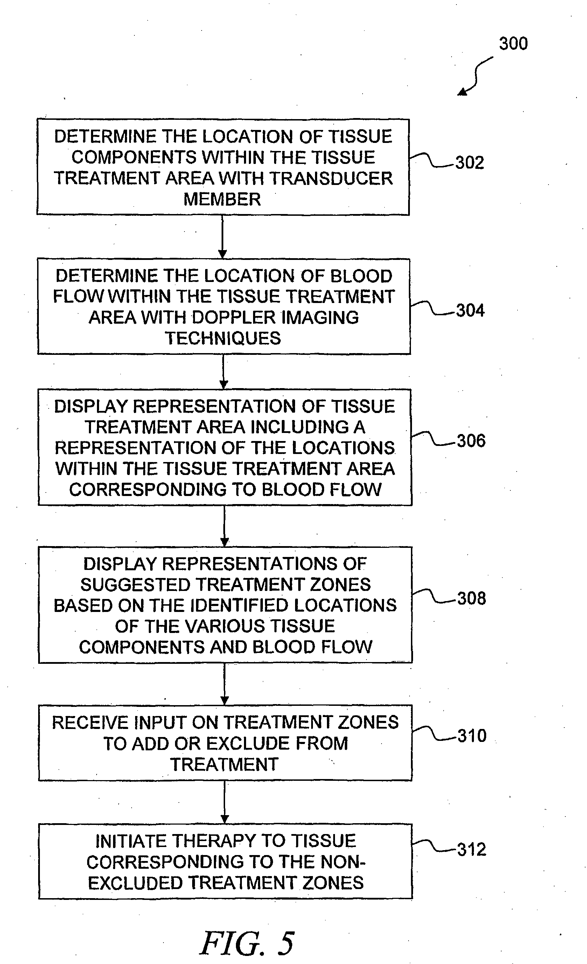

Referring to Fig. 5, an exemplary imaging and treatment method 300 is shown. In a preferred embodiment, imaging and treatment method 300 is carried by controller 108 of HIFU System 100. In one example, imaging and treatment method 300 is embodied in software 109 that is loaded onto memory 111 accessible by controller 108.

In step 302, HIFU System 100 based on ultrasound imaging data collected by transducer member 104 determines the location of tissue components located within the tissue treatment area 10. In one example, HIFU System 100 uses the 3-D modeling techniques discussed herein to determine and model the location of the tissue components, such as rectal wall 18, urethra 14, and prostatic capsule 12.

In step 304, HIFU System 100 based on Doppler imaging data collected by transducer member 104 determines the location, if any, of blood flow in the tissue treatment area 10. In one embodiment, HIFU System 100 uses the 3-D modeling techniques discussed herein to determine and model the location of the tissue components including blood flow. In another embodiment the location of tissue components is determined using both 2-D and/or 3-D imaging and Doppler imaging.

In step 306, HIFU System 100 generates a three-dimensional model of the tissue components in the tissue treatment area including the vascular components which include blood flow. The three-dimensional model is displayed on display 112.

In step 308, HIFU System 100 displays representations or indicia of proposed treatment sites based on the identified location information of the tissue components and/or the location of blood flow in tissue treatment area 10. In one example, HIFU System 100 is configured to identify various treatment zones corresponding to the location of prostatic capsule 12. In one variation, the HIFU System 100 automatically excludes locations which overlap with other tissue components from being suggested or proposed treatment zones, such as locations corresponding to NVB 20 (including blood flow). In another variation, the HIFU System 100 includes locations which overlap with other tissue components as being suggested treatment zones, such as locations corresponding to the neuro-vascular bundles 20. In this variation, the representations or treatment indicia which correspond to these overlapping locations include an indicia or icon differing from the other suggested treatment zones to alert the physician to the location of overlapping tissue (such as a differing color).

In step 310, HIFU System 100 receives input from user input device 110 related to the suggested or proposed treatment zones to add or to exclude. In some instances the physician may decide to proceed with treatment in the area of the NVB 20 to more fully treat the potential diseased tissue and/or because of the patient's wishes. It is important to highlight that the addition of Doppler imaging permits identifying the location of NVB 20, a tissue typically not resolvable by traditional 2-D imaging techniques. As such, the Doppler imaging permits the physician to have location information on the location of the NVB 20 and hence to permit the selective treatment of tissue areas based on the potential damage to the NVB 20.

In step 312, once the treatment zones have been selected and/or approved by the physician, HIFU System 100 focuses high intensity ultrasound energy at the locations in the tissue treatment area corresponding to the treatment zones. As explained more fully in US Patent No. 5,762,066, the high intensity focused ultrasound is an effective tool for selectively destroying diseased tissue surrounded by otherwise healthy tissue in a minimally invasive manner.

As is known HIFU Therapy requires the emission of a continuous wave ("CW") for a sustained period of time with sufficient intensities to ablate the target tissue at the desired location, the focus of transducer 104. For instance, the Sonablate® 500 HIFU system typically is set to provide a CW from its associated transducer for about three seconds resulting in ablation of the target tissue at the focus of the transducer. This time period can be increased or decreased depending on the desired lesion size or the desired thermal dose.

It should be understood that the transducer member 104 of ultrasound system 100 must be capable of being repeatably positioned relative to tissue treatment area in order for the above described method to be effectively carried out. This is because registration is needed between the actual locations of the tissue components and the locations of the suggested treatment zones which are based on the location information derived from the information gathered by transducer member 104. If the transducer member 104 is not capable of being repeatably positioned than there can be no assurance that the location of a treatment zone truly corresponds to the correct location in the tissue treatment area 10.

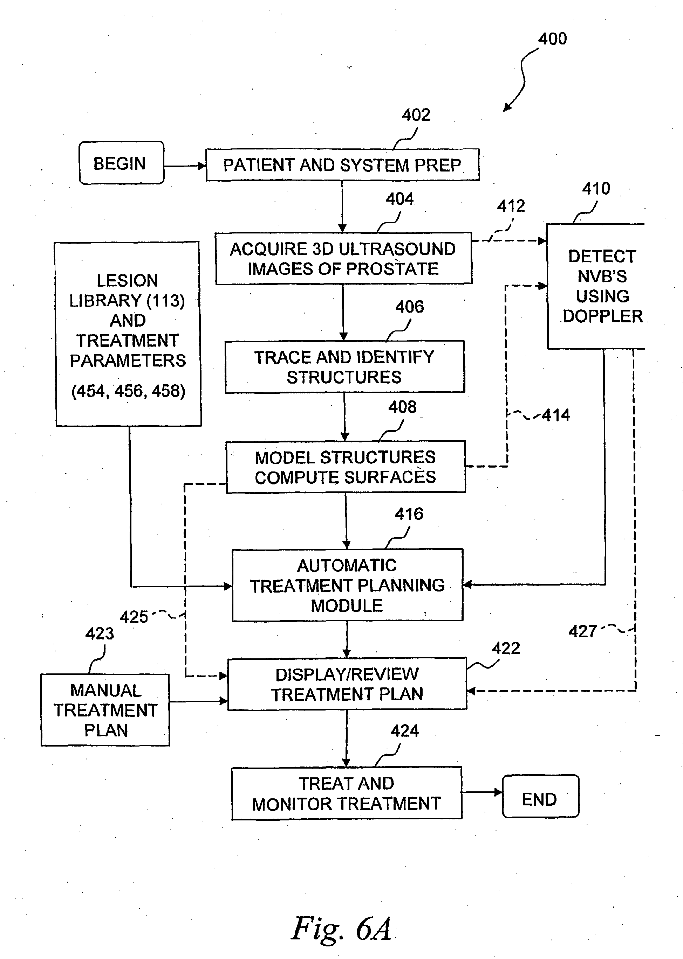

Referring to Fig. 6A, another exemplary method 400 of treating tissue treatment area 10 containing a plurality of tissue components is provided. The exemplary method 400 is tailored to a tissue treatment area including prostate 11 and related tissue components. However, the exemplary method 400 may be used with other tissue treatment areas having other tissue components.

As represented by block 402, the patient and system 100 are setup for the treatment of tissue treatment area 10. In the case of treating prostate 11, the patient and the prostate gland are immobilized. Transducer 104 is positioned proximate to prostate 11 by the transrectal insertion of probe 102 containing transducer 1.04. In one embodiment, the patient is treated under general anesthesia. Probe 102 is held in place by coupling the articulated arm (not shown) to the surgical table (not shown) on which the patient is situated. In one embodiment, images of the tissue treatment area are taken with transducer 104 prior to coupling the arm to the surgical table to verify that the tissue treatment area 10 being imaged with transducer 104 includes the tissue components desired.

The tissue treatment area 10 and hence the patient should remain in the same position relative to probe 102 during the procedure. One reason for this is that the tissue treatment area 10 is imaged and these images are subsequently used to determine the portions of the tissue treatment area 10 which are to receive HIFU Therapy. Assuming that there has not been any appreciable movement between probe 102 and tissue treatment area 10, transducer 104 may be reliably positioned to provide HIFU Therapy to the correct portions of tissue treatment area 10. However, if there has been movement between probe 102 and tissue treatment area 10 then it is not possible to accurately position transducer 104 relative to tissue treatment area 10, and the patient or probe will have to be repositioried or re-aligned prior to treatment planning and HIFU Therapy.

In one embodiment, patient movement is detected by measuring the distance from transducer 104 to rectal wall 18 and to provide an indication of patient movement if that distance changes above a threshold amount. As explained herein, a plurality of sector images and linear images are taken of the tissue treatment area prior to the commencement of treatment with HIFU therapy. Further, immediately after the creation of each individual HIFU lesion (a multitude of these form the overall and complete HIFU treatment), a set of one linear and one sector image are generated (post-lesion images) and displayed with display device 112 along with the associated reference images (stored pre-treatment images) for the same site. By comparing the pre-treatment images and the post-lesion images or features of the pre-treatment images and the post-lesion images patient movement may be detected. In one embodiment, the distance from transducer 102 to rectal wall 18 in the pre-treatment images and post-lesion images are compared to provide one method of detecting patient movement.

As represented by block 404, three dimensional ultrasound images or volume images of the tissue treatment area are obtained. In one embodiment, the volume images are generated based on two-dimensional images of the tissue treatment area. In one embodiment, a plurality of sector images and a plurality of linear images are obtained. Referring to Fig. 2A, an exemplary sector image plane 190 is shown and an exemplary linear image plane 192 is shown. Also shown in Fig. 2A, is an origin 194 of a common probe space 196. The origin is defined by the center of transducer 104 when it is at its lowest position of translation in direction 114 (or the lower most aperture of a scanning aperture transducer) and when transducer 104 is pointing straight out the probe-tip. During the mathematical modeling of the various tissue components discussed herein, various intermediate data-coordinate frames are defined to simplify equations and least-squares fits, but the final equations for the modeled components are transformed back into probe space 196 for display, registration with images, and computer-generation of the treatment plan.

In one embodiment, a plurality of sector images and linear images are obtained. These sector and/or linear images are used to calculate a volume ultrasound image of the tissue treatment area. In one embodiment, the volume image is created by stacking scan-converted two-dimensional sector images. In one example, about 160 sector images, are acquired. Each sector image has 250x357 pixels. The pixel size both in the sector plane and between planes is 0.25 mm, forming cubic voxels (three-dimensional pixels) for distortion-free reconstruction and display. This forms the fundamental 3D prostate imaging dataset. This dataset spans a volume of 40 mm (long (160 pixels)) x 61 mm (height (250 pixels)) x 110° (width (357 pixels)).

Each sector image is inserted into a 3D array in memory 111 for transfer to a rendering board which is incorporated into controller 108. The sector images are equally spaced between the proximal and distal ends of the prostatic capsule, one sector image passing approximately through the middle of the capsule; similarly, the linear images are equally spaced between the lateral limits of the capsule, one linear image passing approximately through the middle of the prostatic capsule.

Controller 108 reconstructs and displays a 3D or volume rendered view of the ultrasound data and treatment zones on display device 112. An exemplary rendering board is VolumePro™500 or VolumePro™1000 available from TeraRecon located at 2955 Campus Drive, Suite 325, San Mateo, CA 94403. In one embodiment, different density tissue in the volume image may be displayed in different colors with the VolumePro ™ 1000. An exemplary volume rendering is shown in Fig. 12. Referring to Fig. 12, the prostate, rectal wall, and fat layer are visible. Further, shown in Fig. 12, are proposed treatment zones for HIFU Therapy. The formation of these proposed treatment zones is discussed herein. In one embodiment, the rendered image via input from user input device 110 may be rotated, may be scaled, and may have different image attributes, such as transparency. With the volume imaging capability, the physician or user is able to view the entire prostate 11 on the screen at one time for pre-treatment and/or post-treatment diagnosis purposes. Further, the volume imaging allows for verification of the planned treatment.

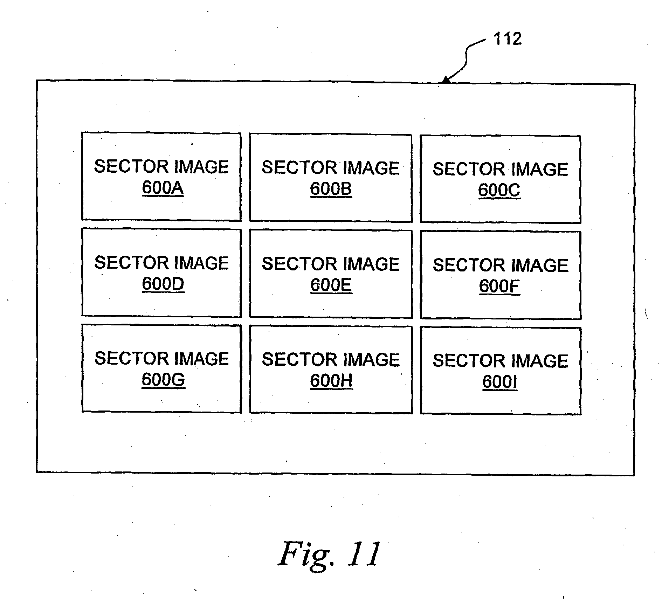

In one embodiment, the user may select to view the tissue treatment area as the volume ultrasound data shown in Fig. 12 or in multiple two-dimensional images, such as a plurality of sector images which have traditionally been used for treatment planning with the Sonablate® 500 HIFU system, such as in Fig. 11 and Fig. 11A.

In one embodiment, the volume image includes interpolated points between the various sector images and/or linear images to enhance the rendered view of the tissue treatment area and/or to reduce the number of sector and/or linear images required. In one example, bilinear interpolation, which ignores data from adjacent planes, is used. In another example, trilinear interpolation is used. In yet another example, a tri-cubic spline, is used.

The volume image data, in one embodiment, is further refined before it is presented to the physician or user for review. One exemplary refinement is adjustments to the histogram of the data to manipulate the contrast and/or brightness of the volume ultrasound data. In one example, the histogram data is set to an "S-shaped" map. Another exemplary refinement is an enhancement of boundaries in the volume data. This enhancement provides assistance to the user in tracing the prostatic capsule 12 and other tissue components in the tracing step, as represented by block 406, below. Further, this enhancement aids controller 108 in identifying the locations of various tissue components, such as rectal wall 18.

As represented by block 406, various tissue components are identified based on the volume image data of the tissue treatment area. At least some of the tissue components, such as rectal wall 18 are identified automatically by controller 108. The rectal wall boundary is automatically detected by simple edge-detection algorithms. In one embodiment, some of the tissue components are identified and located through interaction between a user and the image data, either presented as volume data or as one or more 2-D images. One exemplary tissue component located through user interaction is prostatic capsule 12.

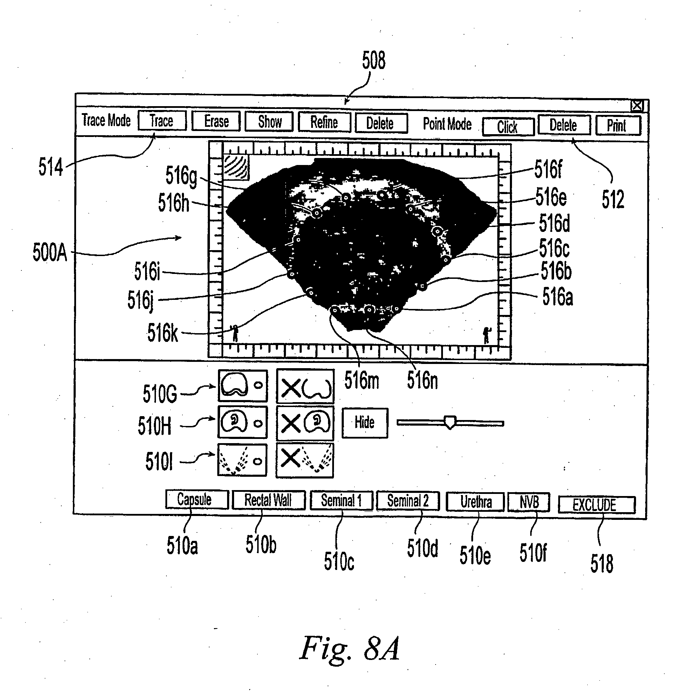

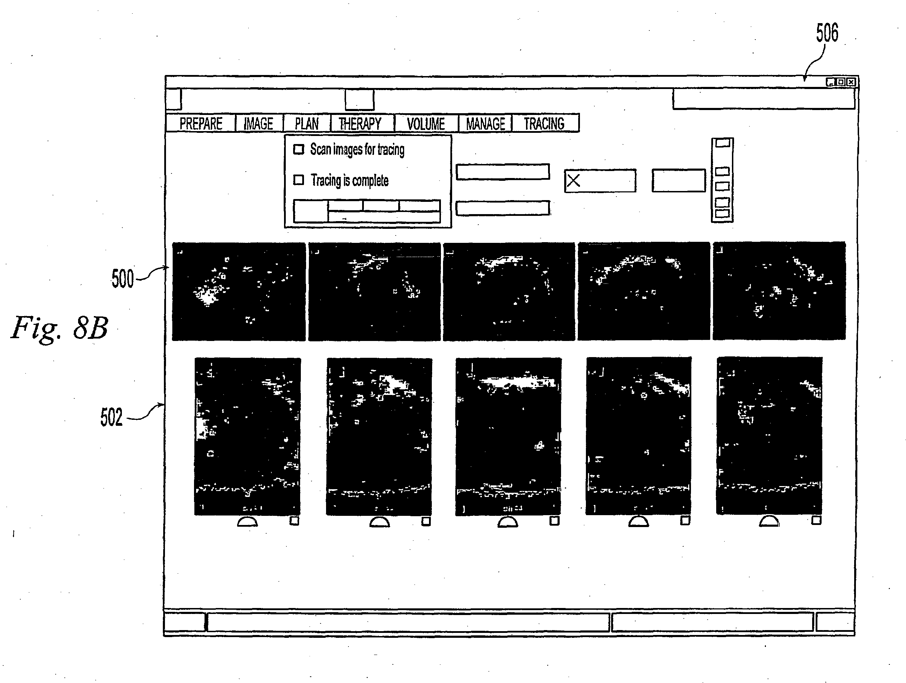

In an exemplary embodiment, illustratively shown in Figs. 8A and 8B, the user is presented multiple sector images 500 and linear images 502 on display 112. The user then traces or otherwise marks the requested components in one or more of the sector images and/or one or more of the linear images. In the case of prostatic capsule 12, the user manually traces the contour of capsule 12 in at least one sector image (illustratively image 500A in Fig. 8A) and manually traces the contour of the capsule 12 at least one linear image. In one embodiment, the user is given up to five sector images and five linear images in which to trace the capsule (see Fig. 8B). In a preferred embodiment, the user traces at least five sector images and at least three linear images. It should be understood that additional tissue components may be traced by the user. In one embodiment, the user marks the center of urethra 14 in each sector image that the user traces capsule 12. In one embodiment, the user traces one or more of urethra 14, seminal vesicles 16, and rectal wall 18, in addition to the prostatic capsule 12.

In one embodiment, for each anatomical structure, the user defines the tracing of a boundary by clicking on points to define a rubber-banding B-spline fit on slices in any or all of the multiple orthographic views. Typically, in the case of prostatic capsule 12 the user will trace the views near mid-gland and at equally spaced intervals to encourage more uniformly spaced data for unbiased geometric model fits.

In addition to tracing the boundary of capsule 12 in each sector image 500, the user is to mark the center of urethra 14 in each sector image 500. In one embodiment, a sonolucent catheter is inserted into urethra 14 during the imaging process to make urethra 16 easier to identify in sector images 500. The catheter is removed prior to HIFU Therapy being administered to the tissue treatment area. The trace data for capsule 12 and urethra 16 are recorded as xyz-Cartesian coordinate data in the probe reference space 196 and written to memory 111 for use in modeling.

An exemplary user interface for tracing or otherwise marking tissue components is shown in Figs. 8A and 8B. The interface 506 of Fig. 8B provides a plurality of sector views 500 and linear views 502 which the user may select to trace boundaries thereto. The interface 508 of Fig. 8A is a trace mode screen wherein the user is presented with an image, illustratively image 500A, on which the user traces or marks appropriate portions of the images 500A. The user is able to select a tissue component to be marked from the plurality of tissue components listed at the bottom of the screen by selecting the corresponding textual button (capsule 510A, rectal wall 510B, seminal vesicles 510C, 510D, urethra 510C, and NVB 510F) or by selecting the corresponding iconic button (capsule 510G, urethra 510H, and seminal vesicles 510I). Further, as shown in Fig. 8A, the user is able to mark components in a point mode 512 and a trace mode 514. In the point mode, the user places points to define the outline of the component (illustratively points 516a-n to define the outline of capsule 12) or the center of the component (in the case of the urethra). Controller 108 then uses the points to generate traces, such as b-spline traces. In the trace mode, the user outlines the region by tracing a closed line (i.e. by holding down a mouse button during the trace). The controller 108 then takes this information to generate a smoother trace.

Further, as explained in more detail below, the user may desire to exclude certain regions of the tissue from treatment. Some of these regions are automatically identified by the system, such as NVB 20 as explained herein. Other regions are identified by the user. One method of identifying these region is to trace or otherwise mark these regions similar to the tracing of tissue components. For instance, the user may select an exclude button 518 and provide trace data for regions that the system should exclude from treatment. One example, may be ejaculatory ducts which are typically not detected by ultrasound, but their location may be inferred by the user from other structures.

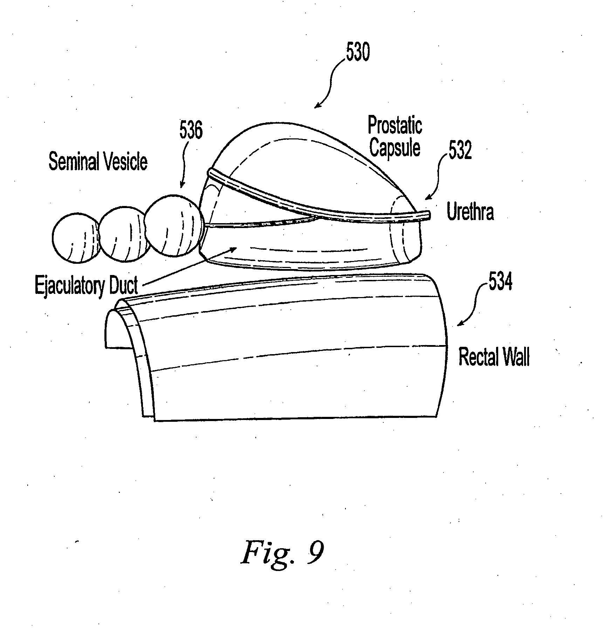

The trace data, other marking data (urethra centers), and automatically located boundaries (such as the rectal wall 18) are used to develop three dimensional models of at least some of the tissue components in the tissue treatment area 10, as represented by block 408. Referring to Fig. 9, the following exemplary models are shown: prostatic capsule 530, urethra 532, rectal wall 534, and seminal vesicle 536. These three-dimensional models are used by system 100 in the automatic generation of a proposed treatment plan, as discussed herein, to assist in the location of other tissue components (such as NVB), to remove clutter from the volume ultrasound data, and/or to provide a visual representation of tissue treatment area 10. Many different techniques may be used to model the tissue components in the tissue treatment area. Exemplary methods of modeling the prostatic capsule 12, the urethra 14, and the rectal wall 18 are provided in the attached APPENDIX and/or in U.S. Provisional Application Serial No. 60/568,556, filed May 6, 2004.

In one embodiment, urethra model 532 is generated by modeling urethra 14 as a parametric tube of the form:

$$x_{urethra}(t,\theta) = h(t) + R\cos\theta \qquad (2a)$$

$$y_{urethra}(t,\theta) = h(t) + R\sin\theta \qquad (2b)$$

$$z_{urethra}(t,\theta) = t \qquad (2c)$$

This circular cylinder is modeled with a constant radius R, such as R = 2.5 mm. This radius size should envelope almost all real urethras. The path h(t) is found by performing a least squares fit to the centers of the urethra identified or marked by the user in the various sector images 500.

In one embodiment, rectal wall model 534 is generated by modeling rectal wall 18 of the form:

$$x_{rectalwall}(t,\theta) = R(t)\cos\theta \qquad (3a)$$

$$y_{rectalwall}(t,\theta) = R(t)\sin\theta \qquad (3b)$$

$$z_{rectalwall}(t,\theta) = t \qquad (3c)$$

Rectal wall 18 is assumed to have a linear axis which corresponds to the probe axis (as indicated in Fig. 2A by direction 114) and a variable radius $R(t)$. The radius function $R(t)$ is determined by performing a least squares fit to the radii of the circles that best fit rectal wall 18 in each sector image 500.

In one embodiment, the left- and right-seminal vesicles are modeled as unions of overlapping spheres of varying radii fit to user-defined boundaries. In order to mark these components the user traces their boundaries, similar to the tracing for the prostatic capsule.

Prostatic capsule 12 may be modeled by various techniques. In one embodiment, prostatic capsule may be modeled as approximating a sphere. In a further embodiment, prostatic capsule 12 may be modeled as an ellipsoid. In another embodiment, prostatic capsule model 530 is generated by modeling prostatic capsule 12 with Fourier ellipsoids. A Fourier ellipsoid is obtained by replacing the (elliptical) cross-sections normal to the major axis of a standard ellipsoid by curves that have a more general Fourier description. This permits the resultant surfaces to have more complex spatially-varying geometric features than standard ellipsoids. This is particularly advantageous when dealing with diseased and/or clipped prostatic capsules whose shape may be irregular.

In one embodiment, the parametric equations for the Fourier ellipsoid are of the form:

$$x_{capsule}(t,\theta) = F(t,\theta)\cos\theta \qquad (4a)$$

$$y_{capsule}(t,\theta) = F(t,\theta)\sin\theta \qquad (4b)$$

$$z_{capsule}(t,\theta) = t, \qquad (4c)$$

where $t \in [-1, 1]$, $\theta \in [0, 2\pi]$, and $F(t, \theta)$ is a truncated Fourier series:

$$F(t,\theta) = (1-t^2)^{\frac{1}{2}}\left\langle f_o(t) + \sum_{n=1}^{N}\left(f_n(t)\cos(n\theta) + g_n(t)\sin(n\theta)\right)\right\rangle, \quad (5)$$

with polynomial blending $f_0(t)$, and $f_n(t)$ and $g_n(t)$, along the linear axis of the capsule. Based on equations 4a-c, the shape of prostatic capsule 12 may be determined along with other parameters such as the volume of prostatic capsule 12, the surface area of prostatic capsule 12, and the relative location of points within the treatment area as being either inside or outside of prostatic capsule 12 or on the surface of prostatic capsule 12. In one embodiment, the surfaces for the prostatic capsule model 530 are used to generate a solid volume model of the prostate. Additional details concerning the modeling of the prostatic capsule are provided in the APPENDIX.

As represented by block 410, Doppler imaging is used to determine and/or assist in determining the location of various tissue components within the tissue treatment area 10. In an embodiment, wherein the prostate is to be treated, Doppler imaging is used to locate NVB 20 so that these nerves may be excluded from treatment. NVB 20 resides close to the surface of prostatic capsule 12 and are densely vascularized. Treatment of NVB 20 with HIFU Therapy may result in impotency, erectile dysfunction, and/or incontinence. However, the user may still decide to treat these regions if the user feels that cancer is in close proximity to NVB 20.

In one embodiment, Doppler imaging data is generated with transducer 104 separate from the generation of the two-dimensional sector and linear images (discussed in relation to block 404). In one example, the two-dimensional sector and linear images are obtained prior to the Doppler information. In one embodiment, as illustrated by dashed line 414 the system uses the location of the shape models to determine portion of the tissue treatment area 10 to scan during the Doppler imaging. This is because NVB 20 are typically in a given spatial relationship to other tissue components such as prostatic capsule 12. Also, in one embodiment, as illustrated by dashed line 412, either the two-dimensional or the volume ultrasound data may provide the location of various tissue components and hence be used to determine the portions of the tissue treatment area to scan during Doppler imaging. As stated herein it is well known in the art to use Doppler imaging techniques to determine the location of blood flow.

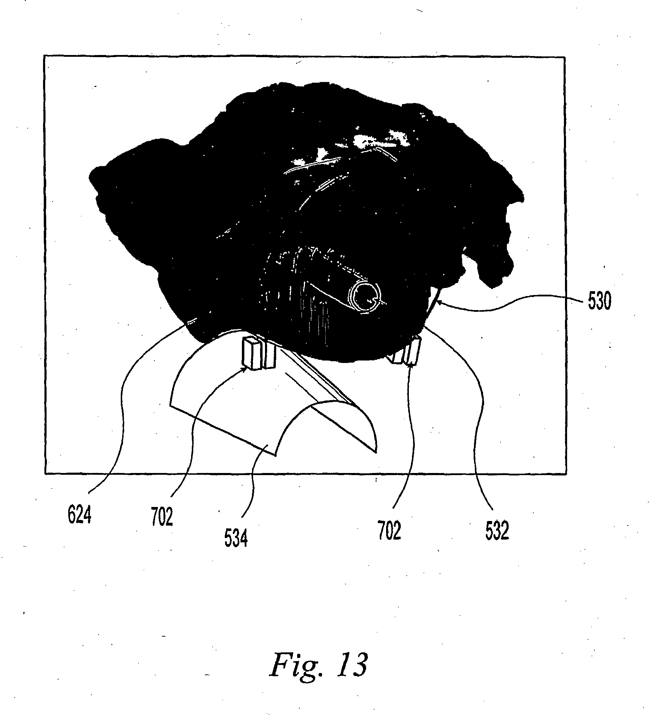

Portions of tissue treatment area 10 exhibiting blood flow may be displayed with display device 112 with a special representation or icon. As shown in Fig. 11, the blood flow may be shown on two-dimensional sector images 600A as icons 602. Further, the blood flow may be shown as an overlay on top of the volume ultrasound image and/or tissue component models via a color-map (see Fig. 13, blood flow icons 702). The color map, in one embodiment color codes the displayed representation or icon to provide an indication of the amount of blood flow present. The display of the blood flow enables the physician or user to visualize the position of the blood flow and its relative position to other tissue components, such as the prostatic capsule (prostatic capsule model 530 in Fig. 13). Further, the physician or user, based on the amount of blood flow present, may be provided an indication of the health of NVB and whether NVB 20 is healthily enough to warrant exclusion from HIFU treatment.

In one embodiment, Doppler imaging is used to detect very small prostate cancer sites. In the early stages of cancer growth the cells form neo-vascularization. Therefore, Doppler imaging may be used to determine the location of these sites. Unlike the case of NVB, these sites are targeted for treatment with HIFU Therapy. In one embodiment, an ultrasound contrast agent is used to enhance the detection of these sites and/or NVB 20.

As explained herein, the Doppler imaging not only provides a visual cue to the user, but it also is one of a plurality of input to an automatic treatment planning module 416 which develops a proposed HIFU treatment plan for review by a user. Traditionally, the Sonablate® 500 HIFU system requires a physician or user to define treatment zones on up to 15 different ultrasound images that span the entire prostate. In one embodiment, HIFU System 100 is configured to generate a proposed HIFU treatment plan consisting of a plurality of treatment zones without the need of input from the user except for desired modifications to the proposed HIFU treatment plan made by the user and the marking of tissue components as discussed herein in connection with block 408.

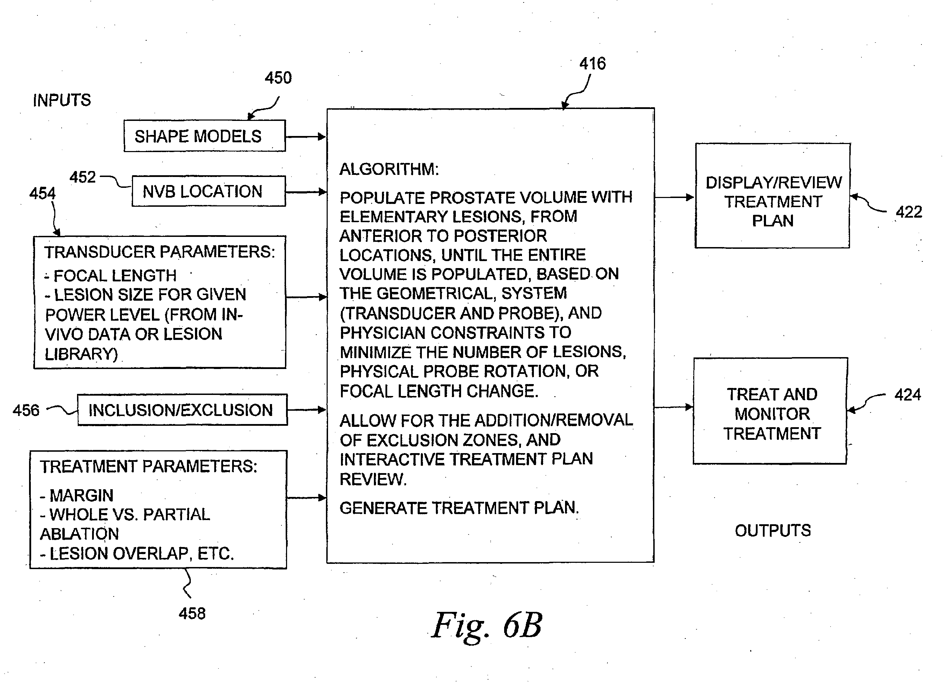

As represented by block 416, an automatic proposed treatment plan is developed to treat portions of tissue treatment area 10. Referring to Fig. 6, the automatic treatment module 416 uses the following five categories of inputs in developing the automatic proposed treatment plan: shape models 450 generated during step 408, location information about the NVB 452 generated during step 410, transducer parameters 454, Inclusion/Exclusion information 456, and treatment parameters 458. Shape models and NVB location information has been discussed above.

Transducer parameters 454 play an important role in the development of an automatic proposed treatment plan. Exemplary transducer parameters 454 include focal length of the transducer, the size of the transducer, and the degree of rotation by the transducer (in one example the transducer may be rotated 110° and still transmit and receive ultrasound energy through a window in the probe housing).

The size and shape of a single thermal lesion produced as a result of HIFU Therapy to a given treatment site is governed by the geometry of the transducer 104 within transrectal probe 102, the duty cycle and repetition rate of the applied acoustic signal, the acoustic properties of intervening tissue types, and the acoustic power delivered at the focus. As explained herein, the present application is not limited to a particular type of transducer 104. However, for illustrative purposes it is assumed that transducer 104 is a spherically focused, truncated spherical shell transducer with a 30 mm diameter aperture and having two transducer faces, one face having a 30 mm focal length operating at 4 MHz and about 30 W of total acoustic power and the other face having a 40 mm focal length operating at 4 MHz and about 37 W of total acoustic power. This is similar to the transducers traditionally used with the Sonablate® 500 HIFU System. Ultrasound exposures for a given HIFU Therapy are assumed to be about 3 sec. HIFU "ON" followed by about 6 sec. HIFU "OFF" duty cycle.

At the transducer and acoustic signal parameters provided (3 Sec ON, 4 MHz, 30W TAP) the dimensions of a single thermal lesion are generally ellipsoidal, approximately 3 mm in width, and approximately 10 mm in length. Further, the thermal lesion is located near the geometric focus of transducer 104. In addition, these elliptical thermal lesions when spaced about 2-3 mm apart tend to merge via thermal diffusion to form a larger necrotic volume. In one embodiment, about 1000 thermal lesions are needed to treat an average human prostate.

The acoustic properties of the intervening tissues are patient specific and temperature dependent. However, the variation in these properties do not substantially change the initial deposition of an isolated thermal lesion from that predicted by an elementary lesion. Thus, the size and shape of a thermal lesion is mainly controlled by the electrical power applied to transducer 104 and the geometry of the transducer, and its subsequent conversion of electrical power to acoustical power.

By varying the focal length of transducer 104, such as with a phased-array transducer, and/or varying the electrical power applied to transducer 104 the size and location of a resultant thermal lesion may be controlled. As such, in areas of the tissue treatment area 10 proximate to critical tissue components, such as the rectal wall 18 or urethra 14, a larger number of low power thermal lesions may be planned compared to other portions of the tissue treatment area 10 such as in the main portion of the prostatic capsule 12. Therefore, detailed treatment plans having pre-defined control of the HIFU dosage (i.e. intensity times exposure time) make it possible to shape thermal lesion patterns (like sculpturing) to increase efficacy and reduce side effects, especially when treating close to rectal wall 18. Also, as explained below these detailed proposed treatment plans may be initially automatically developed and provided to physicians or users for review without requiring the physician to designate treatment sites or zones.

In one embodiment, a lesion library 113 is created wherein lesion size is categorized based on one or more parameters, such as focal length of transducer 104, excitation energy of transducer 104 , HIFU on-time of transducer 104. As such, if the automatic treatment plan module 416 requires a small size lesion, one or more ways of generating such a lesion may be determined based on lesion library 113.

In one embodiment, lesion library 113 is based on in-vivo observations of the size of lesions produced with known parameters, is based on simulated lesions, and/or a combination thereof. In one embodiment, simulation software based on solving the transient bio-heat transfer equation (BHTE), as is well known in the art, is used to simulate various thermal lesions and hence to populate the respective lesion library 113.

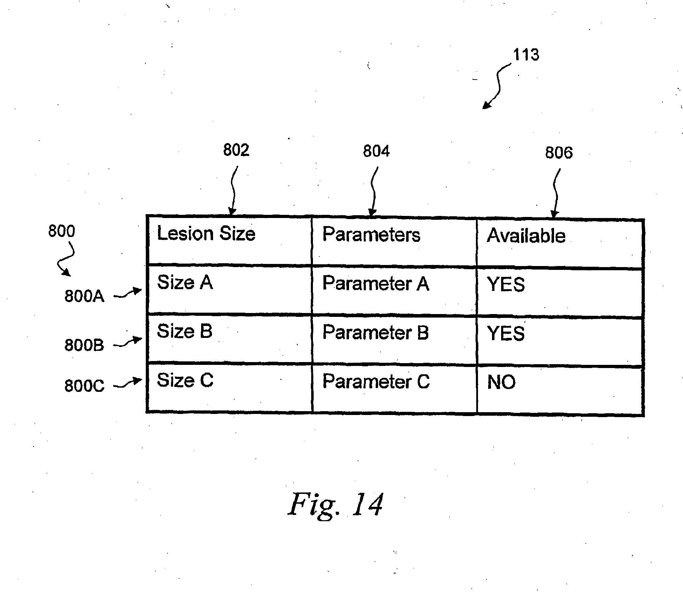

Referring to Fig. 14, a representation of one embodiment of lesion library 113 is shown. Lesion library 113 includes a plurality of exemplary lesions 800, illustratively three 800A-C, which may be used by the automatic treatment module 416 to develop a proposed treatment plan as explained herein. For each lesion 800, a lesion size 802 is provided. Lesion size 802 provides an indication of the expected size of a lesion produced during HIFU Therapy and is based on either in-vivo observations or simulations. In addition, associated parameters 804 are provided for each lesion 800. Parameters 804 are the parameters required to produce the respective lesion 800 and may include transducer, focal length, center frequency of CW, HIFU ON-time, HIFU OFF-time, transducer aperture, power levels, and water standoff distance (the distance between the transducer face and the rectal wall. This distance influences the ultimate size of a lesion as the ultrasound wave is not appreciably attenuated while traveling through water. Thus, for a large water standoff (required to treat close to the rectal wall), larger lesions are generated compared to a small water standoff (required to treat deep in the prostate) using the same power settings). In addition, a status 806 is provided. Status 806 provides an indication whether the respective lesion is available for selection by the automatic treatment module 416. Examples wherein a particular lesion, illustratively lesion 800C, would not be available include situations wherein the transducer used to generate lesion 800C is not currently either coupled to the HIFU System or is otherwise unavailable. For example, lesion 800C may be generated with a 35 mm focal length transducer and only a 30 mm focal length transducer and a 40 mm focal length transducer are available.

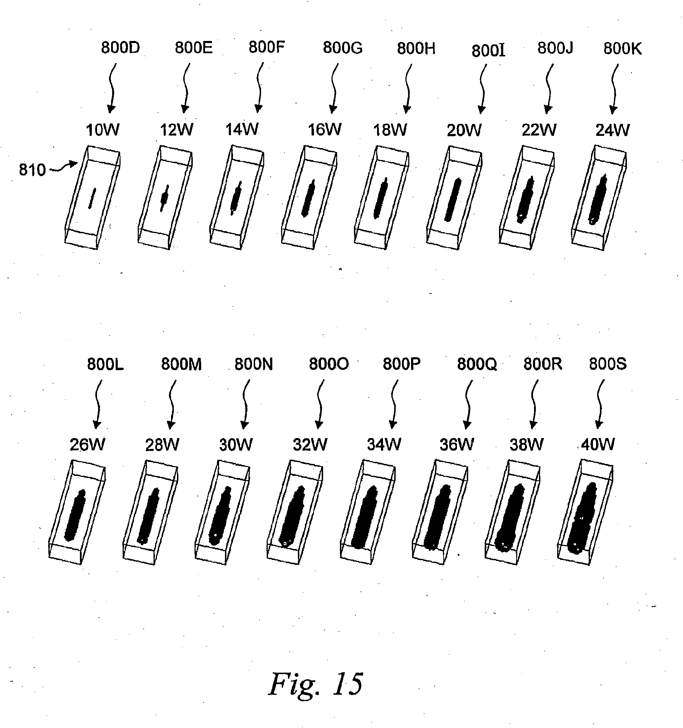

Referring to Fig. 15, representative lesions 800D-S from lesion library 113 are shown. Each of lesions 800D-S are generated with a 40 mm focal length transducer with a 15 mm water standoff. Further, the differences between lesions 800D-S are generated by varying the power level provided at the proposed treatment site. The power level for each lesion 800D-S is provided in Fig. 15 along with a reference box 810 which is the same for each lesion 800D-S and has dimensions of 4X4X12 mm. As shown in Fig. 15, a wide variety of lesion sizes may be generated. As such, lesions 800D-S provide a wide variety of sizes that automatic treatment planning module 416 may select to develop the treatment plan for the tissue treatment region 10.

In addition, various treatment parameters 458 may be defined as an input to the automatic treatment module 416. These treatment parameters are provided by the physician. In one embodiment, the physician is prompted to enter these parameters. One exemplary treatment parameter is margin. Margin is a percentage of a thermal lesion that may cross the boundary of the prostatic capsule into neighboring tissue. Another exemplary treatment parameter is Whole vs. Partial Ablation which relates to whether or not it is desired to treat the entire capsule or only a pre-determined zone, for example. In one embodiment, the predetermined zone may be traced by the physician similar to the capsule. Yet another treatment parameter is Lesion Overlap which relates to the percentage, if at all, the physician desires adjacent treatment sites to overlap each other. Other treatment parameters will be known to those skilled in the art.

These inputs are used to generate an automatic treatment plan for the treatment of diseased tissue in the tissue treatment area 10, such as prostatic capsule 12 in the case of BHP and prostate cancer. The automatically generated treatment plan significantly reduces the overall time required to develop a treatment plan to treat the diseased tissue in the tissue treatment area 10 and provides a valuable decision aid for the physician or user near critical structures, such as rectal wall 18 or NVB 20.

Based on the discussed inputs an automatic treatment plan is generated with the automatic treatment planning module 416. The following discussion assumes that prostatic capsule 12 is to be treated for prostate cancer. However, as stated herein the techniques discussed herein may be used to treat various types of diseased tissues in various tissue treatment areas 10, whether the tissue treatment area includes prostate 11 or not. As such, the disclosed systems 100 and methods should not be limited to only the treatment of prostate 11 and diseases of the prostate.

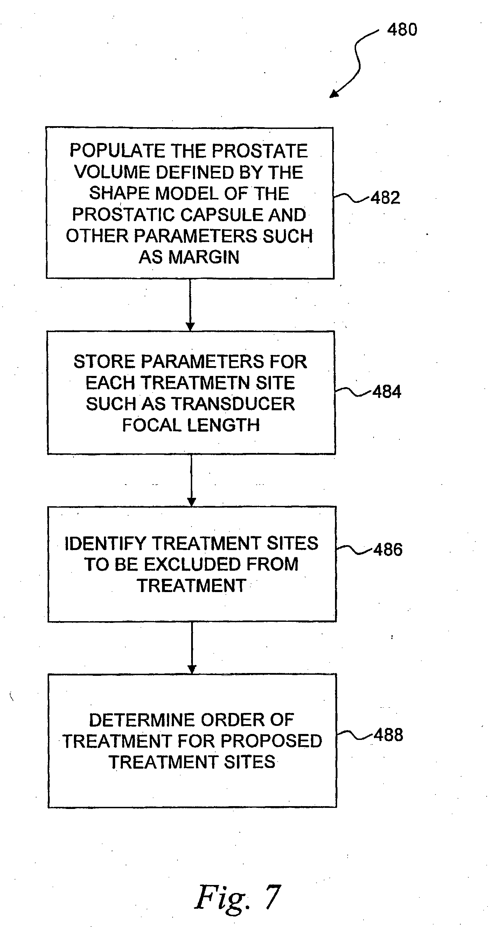

Referring to Fig. 7, an exemplary algorithm 480 for automatic treatment planning module 416 is illustrated. As represented by block 482, proposed treatment sites (proposed locations for thermal lesions) of a given size are positioned such that the entire prostatic capsule model 530 (and slightly beyond the prostatic capsule model 530 based on a value of the margin parameter) is subject to treatment with HIFU Therapy. In a preferred embodiment, the placement of the proposed treatment sites are restricted to portions of the tissue treatment area which were previously imaged during the acquisition of the two-dimensional images as discussed in connection with step 404. In one embodiment, the center of each lesion is proposed to be located at a point in the tissue treatment area 10 which is imaged in at least one sector image and at least one linear image.

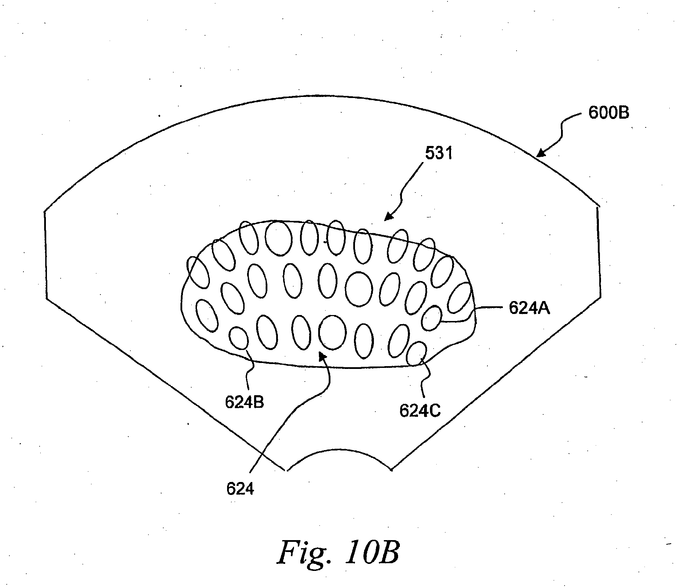

Figs. 10A and 10B show exemplary indicia of proposed treatment sites 624 indicating the exemplary proposed treatment sites for a respective given sector image 600a and 600b. The size and shape of these exemplary indicia of the proposed treatment sites 624 are defined in the lesion library 113. The position of each indicia of proposed treatment sites 624 and hence the position of the resultant actual lesion is determined by the automatic treatment planning module 416. Fig. 10A illustrates generally two radial rows 620 and 622 of treatment sites (for a transducer having two focal lengths) and generally constant size thermal lesions or treatment sites within a given row of treatment sites 620, 622. Fig. 10B illustrates treatment sites 624 at at least three focal lengths and having varying sized lesions. In particular smaller lesions are used to fill in portions near the edge of the prostatic capsule model 530, such as lesion 624a, and proximate to rectal wall 18, such as lesions 624b and 624c. It should be understood that the shown treatment sites do not illustrate the cumulative effect of heating the surrounding tissue. In a preferred embodiment, the spacing of the proposed lesions is chosen such that the individual lesions merge to form a larger lesion.

In one embodiment, larger lesion sizes are proposed where appropriate such as away from the rectal wall 18. By using larger lesion sizes the overall number of lesions may be reduced and hence the overall procedure time is reduced.

In one embodiment, the automatic treatment module 416 develops the proposed treatment sites as follows. First, the parameters provided by the physician are used to determine the area of the prostatic capsule and potentially areas proximate to the prostatic capsule to treat, such as the Margin parameter, and the desired spacing of the lesions, such as the Lesion Overlap parameter.

Once the area of the capsule has been determined, the automatic treatment module 416 selects proposed lesions 800 from lesion library 113. The process is similar to filling a jar with rocks or blocks. First, a gross filling of the jar is completed by placing large rocks or blocks in the jar. Subsequently, a fine filling of the jar is completed by placing smaller rocks or blocks, even sand, in the jar. The smaller rocks or blocks fill in the spaces left open by the large rocks or blocks. In a similar way, the automatic treatment module 416 performs a gross filling of the area to be treated by filling the region with large size lesions 800 from lesion library 113. Next, the automatic treatment module 416 performs a fine filling of the area to be treated by filling the open regions of the area with smaller lesions 800 from the lesion library 113. In contrast to the jar illustration, the automatic treatment module 416 also limits the portions of the area which are filled in the gross filling based on the tissue components in that region, such as near the rectal wall.

As may be appreciated, lesion library 113 provides a plurality of different size blocks or "brushes" for the automatic treatment module 416 to use in planning a proposed HIFU treatment. As explained herein, the size of the blocks or brushes is dependent upon the system parameters including the transducer parameters. In one embodiment, lesion library includes two brushes or blocks, one for a 40 mm transducer at a total acoustic power of 37 watts (W) and one for a 30 mm transducer at a total acoustic power of 20 W. In another embodiment, lesion library has approximately 60 brushes which are generated by varying the total acoustic power (see Fig. 15 for example) for either the 40 mm transducer or the 30 mm transducer or both. In addition, by adding additional transducers to the HIFU system or using a phased array capable of focusing HIFU energy at various focal depths the number of blocks or brushes may be increased.

In addition, to placing the proposed tissue treatment areas system 100 stores the required transducer parameters 454 and/or treatment parameters 458 for each proposed treatment site as represented by block 484. In one embodiment, these parameters are stored in the lesion library 113 along with the size of each lesion 800. Therefore, system 100 determines the required focal length of the transducer 104, the acoustic signal properties, and so forth. These parameters are used during the actual treatment of the tissue treatment area with HIFU Therapy. The automatic treatment module 416 also notifies the user of any additional setup requirements, such as repositioning probe 102 to treat various portions of prostate 11.

As represented by block 486, the automatic treatment plan also automatically removes or deactivates proposed treatment sites based on their proximity to various tissue components such as rectal wall 18 and/or NVB 20. In one embodiment, these treatment sites are not even originally proposed to the physician. In another embodiment, these treatment sites are originally proposed and subsequently deactivated. In one example, the physician selects with user input device 110 the treatment indicia 624 corresponding to the treatment sites the physician desires to remove or deactivate. Further, the physician may select with user input device 110 treatment sites to add to the proposed treatment plan.

As represented by block 488, the automatic treatment plan determines the order of treatment for the proposed treatment sites. In one embodiment, this proposed order of treatment is determined prior to the proposed treatment plan being submitted to a physician or user for review. In another embodiment, the order of treatment for the proposed treatment sites is determined after the physician or user has approved the proposed treatment plan, but prior to commencement of HIFU Therapy to any of the treatment sites. In one exemplary treatment plan, treatment sites in a given sector plane are treated prior to the treatment of treatment sites in another sector plane. Further, treatment sites further from the transducer are treated prior to treatment sites proximal to the transducer (in the case of the prostate anterior to posterior treatment) due to the fact that HIFU therapy changes the properties of tissue in such a way that it prevents tissue ablation behind already ablated tissue.

Returning to Fig. 6, prior to treating the tissue region with HIFU Therapy, the proposed treatment plan is presented to the physician or user for review, as represented by block 422. One example of the presentation of the proposed treatment plane is shown in Fig. 11. In Fig. 11 a plurality of sector images 600a-i are shown with display device 112, each sector image 600 containing a portion of the proposed treatment sites, are displayed with the display device 112. A representation of one of the sector images is shown in Fig. 10A wherein an outline 531 of the shape model 530 for the prostatic capsule is shown, along with the proposed treatment sites shown as a plurality of treatment indicia 624 in rows 620, 622. Further shown are icons or blood flow representations or indicia 602 representing regions of blood flow detected with the Doppler imaging, these regions being associated with NVB 20. As stated herein, in one embodiment, the appearance of representation or indicia 602 indicating the regions of blood flow also include a color indication of the amount of blood flow so that the physician or user may make a determination as to the health of NVB 20. Ultrasound imaging data (not shown) for the given sector is also shown in Fig. 10A. By displaying multiple sector images on the screen at the same time with the automatically generated proposed treatment plan, the physician is able to easily review the proposed treatment plan.

The display shown in Fig. 11 in one embodiment includes buttons, slider controls, or other inputs which permit the selection of the number of sector images to be shown with display. Exemplary arrangements of sector images include 2x2, 3x3 (as shown), and 4x5 matrix display formats. Further controls may be included to select sector slice spacing (0.5, 1, 2, 3, and 3.8 mm), select size and define treatment zones at two different treatment depths, step through a subset of sector slices in higher resolution, and measurement functions (to provide measurements such as from a treatment site to a tissue component such as the urethra). Indicators showing the treatment zones, transducer focal limits, and rectal wall location may be enabled as graphic overlays on each slice (similar to the overlay for the prostatic capsule shown in Fig. 10A).

The physician may also select to view the indicia of the proposed treatment sites 624 as an overlay to the volume ultrasound data. An exemplary representation of such a display is shown in Fig. 12.

The physician also may select to view the proposed treatment sites 624 as an overlay to one or more of the shape models (such as prostatic capsule 530) and/or the volume ultrasound data. The surfaces of the shape models are rendered as thin, almost transparent, shells so the prostate anatomy (volume ultrasound data) and volume treatment zones can be clearly visualized for verification of the treatment plan. In one embodiment, the user is able to interactively select which shape models are to be viewed. In another embodiment, the system 100, limits the amount of volume ultrasound data to be displayed to portions of the tissue treatment area which are inside the prostatic capsule shape model and generally proximate to the outer surface of the prostatic capsule. An exemplary representation of such a display is shown in Fig. 13. In addition, the physician may rotate images on display 112, zoom images on display 112, and slice the images on display 112 through a touch screen input device.

As discussed herein, the physician is further able with user input device 110 to select further regions of the treatment zone for inclusion in the proposed treatment or for exclusion from the proposed treatment. In one embodiment, all of the physician's selections discussed herein are made through a touch screen interface of display 112 and additional input devices 110, such as a keyboard, as needed. Once the physician is satisfied with the proposed treatment plan, the proposed treatment plan is approved, by input from the user (such as selecting a button to approve treatment). Finally, the proposed treatment plan is executed by the system once the order of treatment of the treatment sites has been determined, as represented by block 424.

In one embodiment, automatic treatment planning module 416 is not used or available. In this embodiment, the physician develops a manual treatment plan, as represented by block 423. The manual treatment plan may be laid out by the physician on a plurality of two-dimensional images, such as sector images and/or linear images. The display and review function 422 may still be used to display the manually generated treatment plan and to review the treatment plan. All of the same viewing options are still available with the display of the manual treatment plan, as indicated by line 425 for the shape models and line 427 for the blood flow information. As such, the physician may use the blood flow information and the shape models in the review of the manual treatment plan to determine if modifications are required.

# Modeling Prostate Anatomy from Multiple View TRUS Images for Image-Guided HIFU Therapy

Michael A. Penna*, Kris A. Dines, *Member*,

Ralf Seip, *Member*, Roy F. Carlson, and Narendra T. Sanghvi, *Member*

Abstract- Current planning methods for transrectal high-intensity focused ultrasound treatment of prostate cancer rely on manually defining treatment regions in 15-20 sector transrectal ultrasound (TRUS) images of the prostate. Although effective, it is desirable to reduce user interaction time by identifying functionally related anatomic structures and then automatically laying out treatment sites using these structures as a guide. Accordingly, a method has been developed to effectively generate solid 3D models of the prostate, urethra, and rectal wall, from boundary trace data. Modeling the urethra and rectal wall are straightforward, but modeling the prostate is more difficult and has received much attention in the literature. New results presented here are aimed at overcoming many of the limitations of previous approaches to modeling the prostate while using boundary traces obtained via manual tracing in as few as 5 sector and 3 linear images. The results presented here are based on a new type of surface, the Fourier ellipsoid, and the use of sector *and linear* TRUS images. Tissue-specific 3D models will ultimately permit finer control of energy deposition and more selective destruction of cancerous regions while sparing critical neighboring structures.

*Index Terms* - Image-guided HIFU therapy, prostate, ultrasound, surface model, treatment planning.

## I. INTRODUCTION

Prostate cancer is a major health concern for men. In 2001 it was the leading form of cancer in American men, and in 2004 it was the second leading cause of cancer deaths in American men, second only to lung cancer [2].

For many years, PSA testing and digital rectal exam were the primary methods of diagnosing prostate cancer, and invasive urgery was the preferred method of treatment. Recently, however, several imaging techniques, including transrectal ultrasound (TRUS), have been developed to more accurately diagnose and guide the treatment of prostate cancer, and several non- or minimally invasive techniques have been developed and implemented as clinical systems for treating prostate

Manuscript received 2004; revised ????. This work was partially supported by the National Institutes of Health under SBIR Grant number 2 R44 DK59664.

M. Penna is with the Department of Mathematics, Indiana University-Purdue University, Indianapolis, Indianapolis, IN.

K. Dines is with XDATA Corp. and the Department of Electrical and Computer Engineering, Indiana University-Purdue University, Indianapolis, Indianapolis, IN.

R. Seip is with Focus Surgery, Inc., Indianapolis, IN.

R. Carlson is with Focus Surgery, Inc., Indianapolis, IN.

N. Sanghvi is with Focus Surgery, Inc., Indianapolis, IN.

Human data was collected under approved FDA protocol, and under the IRB and its guidelines, and consent forms were signed prior to collecting data. Collection of data was funded by Focus Surgery, Inc., Indianapolis, IN.

Digital Object Identifier: ????.

Fig. 1. Anatomy of the prostate showing the capsule, urethra, and rectal wall as key structures to be modeled for treatment planning.

cancer. These treatment methods include brachytherapy [11], [17], cryo-surgery [15], external beam radiotherapy [20], and high-intensity focused ultrasound (HIFU) [21]. In HIFU, for example, a high-intensity focused ultrasound beam is used to rapidly heat regions to 70-90°C to kill tissue.

### A. Problem

In each of the above systems, locations within the prostate must be identified for application of the treatment modality. Further, it is often desirable to spare selected regions within and in the vicinity of the prostate from treatment. (It may, for example, be desirable to spare the urethra, the bladder neck, the seminal vesicles, the neurovascular bundles, and the rectal wall, so that normal bodily functions will not be impaired.) Thus there is a need to visualize, identify, and model the prostate and various anatomical structures in and around it, in three dimensions. This paper describes a novel way to do this.

### B. Prostate Anatomy

The prostate is actually a collection of tissues [3], [19].The prostatic capsule (Fig. 1) consists of the central zone, the transition zone, the peripheral zone, and the anterior fibro-muscular stroma. The surface of the capsule is generally smooth, and the capsule is often described as being "nut-like" in appearance.

Other important structures associated with the prostate include the urethra, the seminal vesicles, the neuro-vascular bundle, the bladder and the rectal wall. The capsule has an axial direction which is roughly the same as that of the urethra. The capsule is located close to the rectal cavity, and the rectal cavity is, in fact,

Fig. 2. Transrectal ultrasound imaging and therapy probe.

Fig. 3. The current treatment plan is generated by manually laying out regions at the two focal depths of the 4 MHz ultrasound transducer in 15-20 sector images.

responsible for a shallow indentation on the back of the capsule. The urethra passes from the bladder completely through the capsule. The seminal vesicles, which sit above the capsule, lead to the ejaculatory ducts, and the ejaculatory ducts meet the urethra within the capsule. (The bladder, neuro-vascular bundle, seminal vesicles, and ejaculatory ducts are not illustrated in Fig. .)

### C. Current Treatment Plan

The work presented in this paper arose in the continued development of a commercial TRUS imaging and HIFU therapy system (Sonablate® 500, Focus Surgery, Inc., Indianapolis, IN) whose primary component is a dual-element, confocal ultrasound transducer. One element of the transducer can be used for imaging and the other for the HIFU ablation of tissue. Currently, during a single treatment session the imaging element is used to create both 2D sector (or transverse) and 2D linear (or sagittal) ultrasound scans of the capsule (Fig. 2), treatment zones are defined manually (Fig. 3), and tissue is ablated using the therapy element of the transducer. All of these steps take place while the patient is immobilized on a treatment table, and thus rapid online imaging, modeling, planning, and therapy application are needed to minimize treatment time. (Worldwide, more than 500 men have to date been treated with this system.)

The main disadvantage of the current planning method is that treatment regions have to be manually laid out in 15-20 sector slices, and this takes approximately 1.5 minutes. The proposed planning method requires tracing capsule contours in only 5 sector and 3 linear images, and this part of the proposed method takes only about 1.5 minutes; creation of the model takes about another 1.5 minutes, and laying out the treatment plan geometry (not including dosimetry) takes about 10 seconds. Thus that part of the proposed planning method that is comparable to the current method, takes approximately 3 minutes. In addition, the proposed planning method will allow for automated dosimetry planning, visualization of the treatment plan relative to a model of the capsule and a 3D TRUS image, on a video display device, and more accurate planning at the distal and proximal ends of the capsule. (Dosimetry planning is work in progress and results on it will be reported elsewhere.)

### D. Proposed Treatment Plan

The proposed treatment planning method is based on using a 3D model of the capsule created with contours of the capsule manually traced in sector and linear images (Fig. 4). Two general methods for creating a 3D model of the capsule have been presented in the literature [22]: one involves using manually traced boundary contours in each of several sector images, and the other involves iteratively modifying an initial surface model until some goodness-of-fit criterion is met.

The first method [12], [14], [23], [24] typically involves starting with several manually traced sector scan contours, establishing a correspondence between points on contours in adjacent scans, connecting corresponding points, and filling in the resulting inter-scan grid with some form of surface patch. (Since 2D TRUS images are characteristically speckled and diffuse, and the contrast between the capsule and the surrounding tissue is generally poor, it is not easy to detect the boundary of the capsule in 2D TRUS images. While a great deal of research has been done on automatically identifying the boundary of the capsule in 2D TRUS images [16], manual tracing of the capsule boundary by an expert is still the preferred approach.)

This method has several major disadvantages: It is heavily reliant on the interaction, consistency and accuracy of an expert and it requires contours that are complete and accurate. Further, the models generated are not necessarily unique since they depend on point correspondences between adjacent scans; this dependence is highly noticeable unless large numbers of sector scan contours are used, but manual tracing of a large number of scan contours by an expert is time-prohibitive. Finally, there is no reliable way to model the capsule between its proximal and distal ends and the scans at the respective ends of the sector scan stack, since no data is available there.

The second method [9], [22] has the potential of avoiding the disadvantages of the first. It, however, has the natural disadvantages of being iterative, of requiring proper initialization, and of being based on polygonal models. Piecewise and polygonal models are more sensitive to artifacts due to user-tracing errors, are more difficult to use in generating filled

Fig. 4. With the proposed treatment planning method the capsule is manually traced in up to 5 sector (top row) and 5 linear (bottom row) images.

volume models, and require more complex data structures than the parametric models presented here.

With both methods it is also often assumed that a full view of the capsule is available, so that models can be based on the characteristic shape of the capsule. In practice, however, the entire capsule cannot always be visualized in the limited field of view of a TRUS system. (The probe's limited size, travel and rotation may - as illustrated in Fig. 4 - result in incomplete sector or linear scans.) Further, capsules can be enlarged and distorted due to disease. The assumption that a full view of the capsule is available is another disadvantage to both methods.

The method presented in this paper differs from the above methods in two significant ways. First, the capsule boundary is manually traced not only in several sector scans, but also in several linear scans. These contours - both the sector and linear contours - are used to create a parameterized geometric model of the capsule surface that is then filled to generate a solid model. The linear contours are essential in creating our model: it is usually difficult to identify the capsule in ultrasound sector scans near the proximal and distal ends of the capsule, but it is easy to identify the linear extent of the capsule in linear scans.

Second, our model is created by performing a least-squares fit f a Fourier ellipsoid to contour data. Fourier ellipsoids are smooth, parameterized surfaces that can be specified by only a few parameters, but that can model a large variety of naturally occurring objects, objects such as the surface of a capsule. Fourier ellipsoids are computationally useful since, although they are parameterized surfaces, they enjoy many of the advantages of implicit surfaces: it is easy to determine whether a point is inside or outside such a surface, and it is easy to compute normal vectors to, the surface area of, and the volume enclosed by such surfaces.

In addition to the two general methods for modeling the capsule discussed above, several other methods have been proposed. One involves using models such as the convex hull, that apply to reconstructing objects from good data. Practical considerations limit the usefulness of these methods. Using the convex hull of acquired data, for example, has the disadvantages of not reflecting the true shape of the capsule (specifically the concavity created by the rectal wall) and being unstable (an extraneous outlier can dramatically affect the shape). Another

involves modeling the capsule by ellipsoids or superellipsoids. While this method has the advantage of being simple, it too has the disadvantage of not reflecting the true shape of the capsule (ellipsoids are convex and capsules are generally not). Our method subsumes modeling by ellipsoids and super ellipsoids (since ellipsoids and superellipsoids are Fourier ellipsoids) and it also extends it (since Fourier ellipsoids can be nonconvex). (Ellipsoids and superellipsoids are used in [10], but there their use is intimately tied to automated segmentation. In our work we assume contours are traced manually and hence that segmentation is manual.)

Another approach [7] is to use 2 orthogonal images and scale (or extrude) one against the other. While this approach may allow a coarse approximation of capsule volume, there is no reason to expect that it would lead to a reasonable estimate of shape. Yet another [26] involves building a knowledge base using imaged and excised capsules. The success of this approach is limited by the extent of the knowledge base, by having access to such a knowledge base, and by being able to use it as necessary.

## II. MATERIALS AND METHODS

### A. Data Acquisition

In the proposed treatment plan, 2D sector and linear TRUS images are presented as in Fig. 4. The sector images are equally spaced between the proximal and distal ends of the capsule, one sector image passing approximately through the middle of the capsule; similarly, the linear images are equally spaced between the lateral limits of the capsule, one linear image passing approximately through the middle of the capsule. Currently we model only the capsule, urethra, and rectal wall. The user is expected to manually trace the contour of the capsule and identify the center of the urethra in each sector image, and to manually trace the contour of the capsule in each linear image. The rectal wall boundary is not traced, but automatically detected, because it presents a clean intensity boundary that is easy to identify by simple edge-detection algorithms. (manual tracing is done on a touch-screen, so it is direct and easy for an expert. It takes only about 1.5 minutes to trace 5 sector and 3 linear images of the capsule and urethra.)

Trace data is recorded as $x,y,z$-Cartesian coordinate data in the probe reference space and written to disk for use in modeling. (The modeling of the urethra and rectal wall is discussed in Section IIB, and the modeling of the capsule is discussed in Section IID.) Also written to disk is a 3D TRUS image which is created by interpolating the 2D sector TRUS images (typically at a spacing of between 0.25 and 1mm). As indicated above, the reconstructed model of the capsule is embedded in this image and displayed to provide verification and feedback to the clinician prior to the therapy cycle.

B. *Modeling the Urethra and Rectal Wall*

We model the urethra by a circular cylinder:

$$x(t,\theta) = R\langle\cos\theta,\sin\theta,0\rangle + \langle h(t),k(t),t\rangle$$

that has a constant radius R = 2.5mm and a variable center $C_t(h(t),k(t))$ in each section. The radius was chosen so that the model of the urethra will enclose almost any real urethra, and the coordinates of the center $C_t$ are found by performing a least squares fit to the centers of the urethra identified in the sector images.

We model the rectal wall by a circular cylinder:

$$x(t,\theta) = R(t)\langle\cos\theta,\sin\theta,0\rangle + \langle h,k,t\rangle$$

with a linear axis (the probe axis) and a variable radius R(t). The radius function R(t) is determined by performing a least squares fit to the radii of the circles that best fit the rectal wall in each sector image.

C. *Fourier Ellipsoids*

We model the capsule by a new class of surfaces: Fourier ellipsoids. A Fourier ellipsoid is obtained by replacing the (elliptical) cross-sections normal to the major axis of a standard ellipsoid by curves that have a more general Fourier description. Fourier ellipsoids have more complex spatially-varying geometric features than standard ellipsoids (and are thus amenable to modeling the diseased and clipped capsules) but they are not so complex that they become unwieldy.

The parametric equations for our Fourier ellipsoid in standard form are:

$$x(t,\theta) = a(1-t^2)^{1/2}F(t,\theta)\cos\theta$$
$$y(t,\theta) = b(1-t^2)^{1/2}F(t,\theta)\sin\theta$$
$$z(t,\theta) = ct,$$

where $a$, $b$, and $c$ are constants, $t \in [-1,1]$, $\theta \in [0,2\pi]$, and $F(t,\theta)$ is a truncated Fourier series:

$$F(t,\theta) = f_0(t) + \sum_{n=1}^{N}(f_n(t)\cos n\theta + g_n(t)\sin n\theta)$$

N an integer, $f_0$ (t), and $f_n$ (t) and $g_n$ (t), n $\geq$ 1, being polynomial functions of degree M in t.

For given integers M and N, a Fourier ellipsoid is specified by the (2N + 1) (M + 1) coefficients of the $f_0$ (t), and $f_n$ (t) and $g_n$ (t). Since these constants can be varied continuously, the Fourier ellipsoids associated to any specific integers M and N form a continuously deformable family of parameterized surfaces.

To relate Fourier ellipsoids to other well-known surfaces, observe that if, in (1), $F(t,\theta)=1$ then our surface is the ellipsoid $x^2/a^2 + y^2/b^2 + z^2/c^2 = 1$. If, in (1), we use the constant $r$ instead of $(1-t^2)^{1/2} F(t,\theta)$, then our surface is the right circular cylinder $x^2+y^2 = r^2$. (Thus the effect of including the factor $(1 - t^2)^{1/2}$ in (1) is to smoothly close off the cylinder at $z=\pm1$.) If, in (1), we use a function $f(t)$ independent of $\theta$, instead of $(1-t^2)^{1/2} F(t,\theta)$, then our surface is the surface of revolution $x^2 + y^2 = (f(z))^2$ obtained by revolving the curve $x = f(z)$ about the $z$-axis. (Thus $F(t,\theta)$ can be thought of as the normalized radius of the cross-section at $t$ in the direction $\theta$.) If, in (1), we use $(1 - t^{2n})^{1/2n}$ for some integer $n > 1$, instead of $(1-t^2)^{1/2}$, then we obtain a superellipsoid [5], [81 with more flatness near the north and south poles than an ellipsoid.

If $a = b$ in (1) then for a specific value $\theta_0$ of $\theta$, the intersection of (1) with the plane $y = (\tan \theta_0)x$ through the $z$-axis whose inclination to the $xz$-plane is $\theta_0$, is the (Cartesian coordinate) curve $(t,(1-t^2)^{1/2} F(t,\theta_0)), t \in [-1,1]$. Thus, since a function of the form $f(t) = (1 - t^2)^{1/2} p(t)$, where $p$ is a polynomial in $t$, has vertical tangents at $t = \pm1$, (1) will be smooth where it intersects the $z$-axis; in fact it will have the tangent planes $z = \pm1$ at the points P(0,0,±1), respectively. Further, for specific values of $\theta_0$ (such as $\theta_0 = n\pi/4$, $n = 0,..., 7$), the curves (t, $((1-t^2)^{1/2} F(t,\theta))$ can be thought of as "spines" which, in some sense, define the surface.

If $a = b$ in (1) then for a specific value $t_0$ of $t$, the intersection of (1) with the plane $z = t_0$ is the polar coordinate Curve $(\theta,(1-t_0^2)^{1/2} F(t_0,\theta))$. Thus, since $F(t,\theta)$ is periodic with period (at most) $2\pi$, each cross-section of (1) is a smooth closed curve (with possible self-intersections). In fact, since any function with a finite period can be approximated arbitrarily well by a finite Fourier series, any closed (polar coordinate) curve can be approximated arbitrarily well by a curve of the form $(r(\theta),\theta)$ where $r(\theta)$ is a finite Fourier series. Further, making the Fourier coefficients functions of $t$ allows us to smoothly vary the shape of these cross-sections (as we move up and down the $z$-axis). If $F(t,\theta)= f(t)g(\theta)$ then $(\theta,a(1-t_0^2)^{1/2} f(t_0)g(\theta))$ is the graph of the polar curve $(\theta,g(\theta))$ radially scaled by $a(1-t_0^2)^{1/2} f(t_0)$. Thus a class of Fourier ellipsoids have a natural propensity for cross-sections that are cardioids and limacons.

Fourier ellipsoids are related to Binford's generalized cylinders [1], [4], [6], [13], [18]. Roughly speaking, a generalized cylinder is a parametrized surface of the form

x(t, θ) = A(t) + r(t, θ) cos θ N(t) + r(t, θ) sin θ B(t)

where t ∈ I, θ ∈ [0, 2π], A(t) is a curve in $R^3$ defining the axis of the cylinder, N(t) and B(t) are the unit normal and binormal vectors to A(t), and r(t, θ) is a function that defines the cross-section to the cylinder at A(t). If we let

$$A(t) = \langle 0,0,t\rangle, t \in [-1,1], r(t,\theta) = F(t,\theta),$$ and "close" the cylinder by rescaling in the $x$- and $y$-directions by $(1-t^2)^{1/2}$, then we obtain a Fourier ellipsoid.

We close this section with three observations.

First, even though it is not easy to define an explicit inside-outside function for (1), it is not difficult to determine which points are inside, and which are outside: If, for example, $a = b = c = 1$ then for any point $P(x, y, z)$, let $\theta = \text{Tan}^{-1}(y/x)$ and $t = z$; if:

$$\left(1-t^2\right)^{1/2} F(t,\theta) \begin{cases} < x^2 + y^2 & \text{then P is inside the surface.} \\ = x^2 + y^2 & \text{then P is on the surface.} \\ > x^2 + y^2 & \text{then P is outside the surface} \end{cases}$$

(Inside-outside functions are essential in the generation of the 3D solid models needed for volumetric treatment planning visualization.)

Second, it is easy to compute the surface area:

$$\iint dS = \iint \left\| \left\langle \frac{\partial x}{\partial t}, \frac{\partial y}{\partial t}, \frac{\partial z}{\partial t} \right\rangle \times \left\langle \frac{\partial x}{\partial \theta}, \frac{\partial y}{\partial \theta}, \frac{\partial z}{\partial \theta} \right\rangle \right\| dt \, d\theta$$

and the volume of (1), the later by either voxel-counting or by using the parametrization:

$$X(r, t, \theta) = ar(1-t^2)^{1/2} F(t, \theta) \cos \theta$$
$$Y(r, t, \theta) = br(1-t^2)^{1/2} F(t, \theta) \sin \theta$$
$$Z(r, t, \theta) = ct$$

where $r \in [0,1]$, for the solid capsule. (Surface area and volume are clinically significant quantities.)

Third, while (1) uses $(1-t^2)^{1/2}$ as a flattening function, and $(1-t^{2n})^{1/2n}$, for some integer $n > 1$, could also be used as a flattening function, there are many other possible flattening functions as well. An example is

$$f_\varepsilon(t) = \left(1 - \left(\frac{|t|-1+\varepsilon}{\varepsilon}\right)^2\right)^{1/2}$$

where $0 < \varepsilon < 1$: this function has the value 1 for $t \in [1 - \varepsilon, 1 + \varepsilon]$, and it trails off to 0 at $t = \pm 1$.

### Fitting Fourier Ellipsoids

In this section we address the problem of fitting a Fourier ellipsoid to a set $\{ P_i (x_i, y_i, z_i) \}$ I of data points. One fundamental challenge of this task is that our model is nonlinear and real-time results are required (so iterative techniques, such as the Newton-Raphson Method, are not acceptable). We address this challenge by first finding the center and axes of the (least squares) best fit ellipsoid to our data set. We then fit a Fourier ellipsoid to the data points in the coordinate system whose coordinate axes are these axes. (Our analysis could be simplified if the major axis of the capsule were parallel to the axis of the probe. While this assumption is approximately true, it is not generally true, and tests have revealed that it can lead to inaccurate results. Hence the analysis which we now discuss is necessary.)

To determine the center and axes of our model, we fit a quadric surface of the form
$$Ax^2 + By^2 + Cz^2 + 2Dxy + 2Exz + 2Fyz +$$

$$2Gx + 2Hy + 2Iz + J = 0 \tag{2}$$

to our data. We then orthogonally diagonalize the matrix

$$M = \begin{pmatrix} A & D & E \\ D & B & F \\ E & F & C \end{pmatrix} = PDP^t$$

D is the diagonal matrix of eigenvalues $\lambda_1, \lambda_2, \lambda_3$ of M, and P is the orthogonal matrix ($P^t = P^{-1}$) whose columns are the associated (unit) eigenvectors of M. (To check the shape of the fit we use the fact that (2) is an ellipsoid if and only if $\lambda_1 > 0$, $\lambda_2 > 0$, and $\lambda_3 > 0$.) The equation $(x, y, z) = (u, v, w)P^t$ transforms (2) into the equation of an ellipsoid in $uvw$-coordinates

$$\lambda_1 u^2 + \lambda_2 v^2 + \lambda_3 w^2 + 2gu + 2hv + 2iw + j = 0. \tag{3}$$

which best fits the original data, and the equation $(u, v, w) = (x, y, z)P^t$ transforms our initial data into ellipsoid coordinates. For the sake of argument, we assume this is done in such a manner that the $w$-axis is the major axis of the ellipsoid, and we rewrite (3)

$$\frac{(u-u_0)^2}{a^2} + \frac{(v-v_0)^2}{b^2} + \frac{(w-w_0)^2}{c^2} = 1$$

where $a^2 = 1/\lambda_1$, $b^2 = 1/\lambda_2$, and $c^2 = 1/\lambda_3$.

We now translate our data so that $(u_0, v_0, w_0)$ becomes the data center, and we rescale our data by $1/a$, $1/b$, and $1/c$ in the $u$, $v$, and $w$ directions, respectively. Having done this, however, the coordinates of our model in standard form are still not exactly uvw-coordinates: Since we wish to flatten our model at its ends using $(1-t^2)^{1/2} = (1-w^2)^{1/2}$ we must first translate the data in the $w$-direction so that the origin is exactly half way between the data points $(u_i, v_i, w_i) = (x_i, y_i, z_i)P$ for which $w_i$ is largest and smallest, and rescale the coordinates of each data point by:

$$k = (\max w_i - \min w_i)/2$$

(replacing $(u_i, v_i, w_i)$ by $(u_i/k, v_i/k, w_i/k)$).

Now we compute the $(r, \theta, t)$-cylindrical coordinates of each data point $(u_i, v_i, w_i)$: having assumed the $w$-axis is the major axis of the capsule, $r_i = \left(u_i^2 + v_i^2\right)^{1/2}$, $\tan \theta_i = v_i/u_i$, and $t_i = w_i$. Assuming that

$$f_n(t) = \sum_{m=1}^{M} f_{nm} t^m \text{ and } g_n(t) = \sum_{m=1}^{M} g_{nm} t^m, \text{ for some}$$

constants $f_{nm}$ and $g_{mn}$, we obtain a linear equation in the $f_{nm}$'s and $g_{mn}$'s for each data point:

$$r_i = (1-t_i^2)^{1/2} F(t_i, \theta_i) =$$

$$(1-t_i^2)^{1/2} \sum_{n=0, m=1}^{N,M} \left( f_{nm} t_i^m \cos n\theta_i + g_{nm} t_i^m \sin n\theta_i \right)$$

and the resulting system is easily solved.

Having to translate the origin prior to the fitting process is not a significant consideration since it occurs along the main axis of the data while fitting is done axially (in cylindrical coordinates) about this axis. In fact, if we chose to use a flattening function whose value is 1 near mid-gland (such as the function mentioned at the end of Section IIC) then there is absolutely no mid-gland

effect of translation (although data would still be flattened at the proximal and distal ends of the capsule).

Fig. 5. Three views of an ideal capsule.

Fig. 6. Fitting the ideal capsule (nS = 5, nL = 3, M = 3, N = 3).

Fig. 7. Fitting the ideal capsule (nS = 15, nL = 0, M = 3, N = 3).

## III. RESULTS

The modeling process presented above was implemented using a compiled Matlab® (MathWorks, Inc., Natick, MA) program called by the Java user interface of the Sonablate® 500. The modeling of the urethra and rectal wall is straightforward; in this section we focus on the modeling of the capsule. Tests were performed using synthetically generated data and real in vivo data from a phantom, dogs, and human subjects.

### I. Synthetic Data

Our synthetic implementation was performed in parallel with, and guided by, our real implementation. Thus, for example, our synthetic models were created in a virtual 3D TRUS image that was (like a real 3D TRUS image) nx x ny x nz = 280 x 280 x 160 voxels, each voxel (like a real voxel) being dx x dy x dz = 0.25 x 0.25 x 0.25 mm3 in size. Further the axis of the probe was (like the probe in a real 3D TRUS image) assumed to go from (nx, ny/2, 1) to (nx, ny/2, nz) in the z-direction.

We created more than 24 ideal capsules and corresponding trace sets, each trace set consisting of nS sector and nL linear traces. Three images of a typical capsule taken from different viewpoints are shown in Fig. 5.

First some qualitative results.

Assuming we use the flattening function (1 -t2)1/2 in (1), there are four parameters necessary to fit a model to trace data: nS, nL, M, and N. Fig. 6 illustrates the results of using nS = 5, nL = 3, M = 3, and N = 3 in reconstructing the ideal capsule illustrated above. We found nS = 5, nL = 3, M = 3, and N = 3 are good alues to use in general: Using nS = 5 and nL = 3 keeps the number of traces the expert is required to trace small, but produces reasonably accurate real results. Using M = 3 and N = 3 produced very accurate real results; larger values of M and N did not produce significantly better results, and they did tend to result in over-fitting and singular matrices.

Further tests using nS = 5, nL = 3, M = 3, and N = 3 confirmed that our method does not depend on the orientation of the capsule relative to the probe.

Fig. 7 illustrates the results of using nS = 15, nL = 0 (no linear traces), M = 3 and N = 3 in reconstructing the capsule. This figure illustrates that while a large number of sector contours can generate a good model of the capsule at mid-gland, linear traces are absolutely necessary in creating a good model of the capsule at its proximal and distal ends.

Fig. 8 illustrates an ideal capsule clipped laterally and longitudinally, Fig. 9 illustrates the results of using nS = 5, nL = 5, M = 3, and N = 3 to model the laterally clipped capsule, Fig. 10 illustrates the results of using nS = 5 nL = 5, M = 3 and N = 3 to model the longitudinally clipped capsule, and Fig. 11 illustrates the results of using nS = 5, nL = 5, M = 5, and N = 5 to model the laterally and longitudinally clipped capsule. In general, we found that that our modeling responded well to reasonable amounts of clipping. While we found that with lateral or longitudinal clipping we obtained good results with M = 3 and N = 3, we found that we obtained good models with laterally and longitudinally clipping when M and N were increased slightly.

Now some quantitative results. It is difficult to compare our model to any of the models discussed in Section ID

Fig. 8. A clipped ideal capsule.

Fig. 9. Fitting the laterally clipped ideal capsule.

ig. 10. Fitting the longitudinally clipped ideal capsule.

because of the reasons cited there, except for the ellipsoid and superellipsoid models. Since the comparison between the ellipsoid model and the Fourier ellipsoid model whose flattening function is $(1 - t^2)^{1/2}$ is comparable to the comparison between the superellipsoid model and the Fourier ellipsoid model whose flattening function is $(1 - t^{2n})^{1/2n}$, and since space is limited, we restrict our attention to the former comparison.

We created 6 cubic spline *(not* Fourier ellipsoid) capsule models, and corresponding trace sets, each trace set consisting of $nS = 5$ sector and $nL = 3$ linear traces. We fit ellipsoid models and Fourier ellipsoid models to these trace sets, $M$ and $N$ for the Fourier ellipsoid models going from $M = 2$ and $N = 2$ to $M = 4$ and $N = 8$. For a typical full capsule, the ellipsoid model had an accuracy of 84.13%, with false positives of 16.65% and false negatives of 15.87%; the average accuracy for the Fourier ellipsoid model (the average computed over the range for $M$ and $N$ mentioned above) had an accuracy of 95.78%, with false positives of 3.03% and false negatives of 4.22% (see Table 1). By Accuracy we mean the ratio of the volume of overlap of the reconstructed model and the original capsule, to the volume of the original capsule. By False Positive we mean the ratio of the volume of that part of the reconstructed model that lies outside the original capsule, to the volume of the original capsule. By False Negative we mean the ratio of the volume of that part of the

Fig. 11. Fitting the laterally and longitudinally clipped ideal capsule.

original capsule that lies outside the reconstructed model, to the volume of the original capsule.) For a typical truncated capsule (a capsule truncated 20% in the $z$-direction at its distal end and 20% in the $z$-direction at its proximal end), the ellipsoid model had an accuracy of 77.98%, with false positives of 20.96% and false negatives of 22.02%; the average accuracy for the Fourier ellipsoid model (the average again being computed over the range for $M$ and $N$ mentioned above) had an accuracy of 93.86%, with false positives of 3.96% and false negatives of 6.14% (again see Table 1).

| N | M | Full Capsule | | | Truncated Capsule | | |
|---|---|---|---|---|---|---|---|
| | | Accuracy | False Pos. | False Neg. | Accuracy | False Pos. | False Neg. |
| 2 | 2 | 94.64 % | 3.91% | 5.36 % | 90.99 % | 6.81 % | 9.01 % |
| 2 | 3 | 94.75 % | 4.05 % | 5.25 % | 91.03 % | 6.88 % | 8.97 % |
| 2 | 4 | 94.87 % | 4.07 % | 5.13 % | 91.48 % | 6.69 % | 8.52 % |
| 3 | 2 | 95.42 % | 3.29 % | 4.58 % | 92.35 % | 5.45 % | 7.65 % |
| 3 | 3 | 95.55 % | 3.30 % | 4.45 % | 92.41 % | 5.42 % | 7.59 % |
| 3 | 4 | 95.70 % | 3.25 % | 4.30 % | 92.98 % | 5.08 % | 7.02 % |
| 3 | 2 | 95.84 % | 2.97 % | 4.16 % | 94.01 % | 3.43 % | 5.99 % |
| 4 | 3 | 96.00 % | 2.93 % | 4.00 % | 94.15 % | 3.41 % | 5.85 % |
| 4 | 4 | 96.12 % | 2.79 % | 3.88 % | 94.90 % | 2.98 % | 5.10 % |
| 4 | 2 | 95.92 % | 2.85 % | 4.08 % | 94.11 % | 3.45 % | 5.89 % |
| 5 | 3 | 96.09 % | 2.87 % | 3.91 % | 94.19 % | 3.39 % | 5.81 % |
| 5 | 4 | 96.19 % | 2.61 % | 3.81 % | 94.97 % | 2.94 % | 5.03 % |
| 6 | 2 | 96.03 % | 2.84 % | 3.97 % | 94.21 % | 3.36 % | 5.79 % |
| 6 | 3 | 96.18 % | 2.84 % | 3.82 % | 94.28 % | 3.35 % | 5.72 % |
| 6 | 4 | 96.06 % | 2.58 % | 3.94 % | 95.18 % | 2.85 % | 4.82 % |
| 7 | 2 | 96.05 % | 2.74 % | 3.95 % | 94.54 % | 3.18 % | 5.46 % |
| 7 | 3 | 96.17 % | 2.81 % | 3.83 % | 94.63 % | 3.18 % | 5.37 % |
| 7 | 4 | 95.89 % | 2.57 % | 4.11 % | 95.64 % | 2.60 % | 4.36 % |
| 8 | 2 | 96.01 % | 2.83 % | 3.99 % | 94.64 % | 3.08 % | 5.36 % |
| 8 | 3 | 96.09 % | 2.87 % | 3.91 % | 94.72 % | 3.10 % | 5.28 % |
| 8 | 4 | 95.82 % | 2.67 % | 4.18 % | 95.73 % | 2.57 % | 4.27 % |

Table 1: Synthetic implementation results *(nS* = 5 and *nL* = 3).

Since, as indicated in Section IIB, ellipsoids are Fourier ellipsoids, these results are not surprising: any fit by Fourier ellipsoids can be no worse than a fit by an ellipsoid.

To quantify the need for using linear traces, we studied the case in which $nS$ is arbitrary and $nL = 0$. We found that for a full capsule for which $nz = 160$ (that is which has 160 horizontal slices), if $nL = 0$ then $nS$ must be at least 140 - in other words, at least 140 slices must be traced - in order to achieve an accuracy of 95%.

We did not test our method by adding white noise to data since, when dealing with real data, other factors (such as specularity of images and accuracy of manual traces) far outweigh the effects of white noise.

*B. In vivo Data*

Results were obtained using data from a phantom, 2 dog, and 10 human subjects who were treated for prostate cancer. (For some patients only one data set was collected; for some patients more than one data set was collected to allow us to compare different data sets for the same person.) While we can provide synthetic evaluation of our model, quantitative in vivo validation is not possible for several reasons. First, as far as the phantom was concerned, the phantom to which we applied our method was encased, and it was - and still is - being used in the development of the treatment system reported on in this manuscript; destroying this phantom was impossible. Second, as far as dogs and humans are concerned, clinical protocols for the system we are working on prohibit excision of the gland post-treatment: since our system

is a priori non-invasive, treated capsules must of necessity remain in vivo to determine the efficacy of non-invasive surgery. And

Fig. 12. HSI *with nS=5,nL=3, M=3, N=3.*

Fig. 13: HS2 with *nS = 3, nL = 5, M = 3, N = 3.*

even if we were to work with capsules excised post-mortem (or even post imaging), the effects of time, treatment, and the excision process itself (all of which can distort the shape of the capsule) stand in the way of ever establishing any form of ground-truth or gold-standard. The best we could hope for - and what we were happy with in the end - was when experts consistently viewed the results of our modeling in a 3D volume display and called them reasonable.

Preliminary tests with data from the phantom, dogs, and humans illustrated that while the advantage to using large values of $M$ and $N$ is that more shapes are available, the disadvantage is the tendency to overfit data and the fact that the matrix used to find the constants $f_{nm}$ and $g_{nm}$ can be singular. On the other hand, while the advantage to using small values of $M$ and $N$ is more control over shapes (and there is less of a tendency to overfit data), the disadvantage is that shapes are restricted and may not reflect pathology.

The entire capsule of human subject 1 (HS1) was visible in the field of view of the TRUS system, and exhibited the classic capsule shape. Fig. 12 shows the surface generated by using 5 sector and 3 linear traces. In this case the surface fit conformed well to the trace data and the linear traces clearly helped define the proximal and distal ends of the capsule.

The full capsule was again visible in human subject 2 (HS2). The shape, shown in Fig. 13, generated using 3 sector and 5 linear traces was again judged by clinical experts to conform to their mental reconstruction obtained by viewing the sector and linear images during examination. Full and cut-away views of the capsule, urethra, and rectal wall for HS2 are shown in Fig. 14. A solid 3D model was generated by filling the interior of the surfaces, and a preliminary automatic treatment plan was then generated for one sector slice as shown in Fig. 15. The HIFU lesion sites are distributed in the capsule with the aim of avoiding the urethra (shown as a small white circle). The model of the rectal wall (rectal cavity) is used to compute the ultrasound

attenuation along the path to sites and hence the energy deposition required for each site.

Fig. 14. Full and cut-away views of the capsule, urethra, and rectal wall for HS2.

Fig. 15. A preliminary treatment plan generated for one sector slice of HS2.

Computation of the capsule surface models and generation of the capsule solid models required about 90s on a Pentium 4 at 2.8 GHz. Of this total time only about 10s was required to model the surface and the rest was consumed by the filling algorithm in generation of the solid model.

The capsule was clipped for human subject 3 (HS3). The traces for HS3 are shown in Fig. 4. The model of HS3 shown in Fig. 16 illustrates the flexibility of the Fourier ellipsoid model in adapting to shapes that are far from true ellipsoids.

## IV. CONCLUSION

In this paper we have presented a new method for modeling the prostatic capsule, urethra and rectal wall, in three dimensions.

In comparison to current methods for modeling the capsule, our approach involves smooth parametric models that enjoy many of the benefits of implicit surfaces (while not suffering from the drawbacks of implicit surfaces), it requires specification of only four integer parameters *(nS, nL, M,* and *N)* to determine a unique model, it does not demand great accuracy in manually tracing scan contours, nor does it require complete contour information (since it is a least squares technique with which surfaces are fit to 3D points, not 2D contours). Our method does not depend on the orientation of the capsule relative to the probe, it is stable in the presence of noise and outliers, it assumes only

Fig. 16.HS3 with *nS = 5, nL = 3, M = 3, N = 3.*

that the surface of the capsule is smooth and that it approximates the sector and linear scan contours, and it works well with "clipped" capsules. It is and deterministic (not iterative) and typically requires traces in only 5 sector and 3 linear scans to yield good results; further these good results are good not only at mid-gland, but also at the proximal and distal. ends of the capsule since there is a natural closure to our models which is supported at the ends of the capsule by data in the linear slices. The drawbacks of our method are that traces of sector and linear boundary views must be consistent with actual boundaries (the accuracy of our models relies on the accuracy of the experts doing the manual tracing), and that linear views are not naturally available on most TRUS systems (but must be extracted as planar sections of reconstructed 3D TRUS images).

Our method has the potential of allowing tissue to be identified by type, and knowing the spatial distribution of tissue by type is desirable in computing the amount of energy deposition required destroy tissue, since doing so improves the control of therapy. Ongoing research is directed at automatic segmentation of the capsule in 2D and 3D TRUS images, identifying tissue by type, and fully automated treatment planning.

## ACKNOWLEDGMENT

The authors would like to thank Dr. Russ Fedewa of Focus Surgery, Inc., Indianapolis, IN, for the preliminary treatment planning figure, as well as Dr. Thomas Gardner, MD, and Dr. Michael Koch, MD, both of the Indiana University School of Medicine, for their great help in the acquisition of human 3D ultrasound prostate data.

## REFERENCES

[1] G. Agin and T. Binford, "Computer description of curved objects", IEEE Trans. Computers, vol. C-25, no. 4, pp. 439-449, April 1976.

[2] American Cancer Society website: http: //www. cancer. org.

[3] The Prostate Gland, Anatomical Chart Co., Skokie, Illinois.

[4] D. Ballard and C. Brown, Computer Vision, Englewood Cliffs, NJ: Prentice-Hall, Inc., 1982.

[5] Barr, "Superquadrics and angle preserving transformations", IEEE Computer Graphics and Applications, pp. 11 -23, Jan. 1981.

[6] V Burdin and C. Roux, "Modeling and analysis of 3-D elongated shapes with applications to long bone morphometry", IEEE Trans. Medical Imaging, vol. 15, no. 1, pp. 79-91, Feb. 1996.

[7] D. Crivianu-Gaita, F. Miclea, A. Gasper, D. Margineatu, and S. Holban, "3D reconstruction of prostate from ultrasound images", Int J Medical Informatics, vol. 45, pp. 43-51, 1997.

[8] W. Franklin and A. Barr, "Faster calculation of superquadric shapes", IEEE Computer Graphics and Applications, pp. 41-47, Jan. 1981.

[9] Ghanei, H. Soltarian-Zadehm A. Ratkewicz, and F. F. Yin, "A three-dimensional deformable model for segmentation of human prostate from ultrasound images", Med. Phys., vol. 28. no. 10, pp. 2147-2153, Oct. 2001.

[10] L. Gong, S. D. Pathak, D. R. Haynor, P. S. Cho, and Y. Kim, "Parametric shape modeling using deformable superellipses for prostate segmentation", IEEE Trans. Medical Imaging, vol. 23, no. 3, March 2004.

[11] L. Gunderson, Intraoperative Irradiation: Techniques and Results, Totowa, NJ: Humana Press, 1999.

[12] W. C. Lin and S. Y. Chen, "A new surface interpolation technique for reconstructing 3D objects from serial cross-sections", CVGIP, vol. 48, pp. 124-143, 1989.

[13] R. Nevatia and T. Binford, "Description and recognition of curvedobjects", Artificial Intelligence, vol. 8, pp. 77-98, 1977.

[14] O. S. Odesanya, W. N. Waggenspack, and D. E. Thompson, "Construction of biological surface models from cross-sections", IEEE Trans Biomedical Engineering, vol. 40, no 4, pp.329-334, April 1993

[15] G. M. Onik, J. K. Cohen, G. D. Reyes, B. Rubinsky, Z. Chang, and J. Baust, "Transrectal ultrasound-guided percutaneous radical cryosurgical ablation of the prostate", Cancer, vol. 72, 9. 1291-1299, 1993.

[16] S. D. Pathak, V. Chalana, D. R. Haynor, and Y. Kim, "Edge-guided ablation of the prostate", Cancer, vol. 72, pp. 1291-1299, 1993. boundary delineation in prostate ultrasound images", IEEE Trans. Medical Imaging, vol. 19, pp. 1211-1219, 2000.

[17] Z. Petrova, Carcinoma of the Prostate: Innovations in Management, Berlin, New York: Springer, 1996.

[18] J. Ponce, D. Chelberg, and W.B. Mann, "Invariant properties of strait homogeneous generalized cylinders and their contours", IEEE Tran. PAMI, vol. 11, no. 9, pp. 951-966, Sept. 1989

[19] R. Rhodes and R. Pflanzer, Human Physiology 3rd Ed., Fort Worth, TX: Saunders College Pub., 1996.

[20] R. H. Sagerman, H. C. Chun, G.A. King, C.T. Chung, and P.S. Dalal, "External beam radiotherapyfor carcinoma of the prostate", Cancer, Vol.63, pp. 2468-2474.

[21] N. Sanghvi, R. Foster, R. Bihrle, R. Casey, T. Uchida, M. Phillips, J. Syrus, A. Zaitsev, K. Marich, and F. Fry, "Noninvasive surgery of prostate tissue by high intensity focused ultrasound: an updated report", European Journal of Ultrasound, vol. 9, pp. 19-29, 1999.

[22] F. Shao, K. V. Ling, W. S. Ng, and R. Y. Wu, "Prostate boundary detection for ultrasonographic images", J. of Ultrasound in Medicine, vol. 22, no. 6, pp. 605-623, June 2003.

[23] L. L. Shumaker, "Reconstructing 3D objects from cross-sections", Computation of Curves and Surfaces, W. Dahmen et al eds., New York: Kluwer Academic Publishers, pp. 275-309, 1990.

[24] P. N. Werahera, G. H. Miller, G. D. Taylor, T. Brubecker, F. Daneshgari, and E. D. Crawford, "A 3D reconstruction algorithm for interpolation and extrapolation of planar cross sectional data", IEEE Trans. Medical Imaging, vol. 14, no. 4, pp. 765-771, Dec. 1995.

[25] R. Y. Wu, K. V. Ling, and W. S. Ng, "Automatic prostate boundary recognition in sonographic images using feature model and genetic algorithm", J Ultrasound in Medicine, vol. 19, pp. 771-782, 2000.

[26] J. Zeng, J. J. Bauer, and S. K. Mun, "Modeling and mapping of prostate cancer", Computers & Graphics, vol. 24, pp. 683-694, 2000.

## Claims

1. An apparatus for treating a tissue treatment area including a plurality of tissue components, the apparatus comprising a transducer (104) which is positionable relative to the tissue treatment area (10), the transducer (104) being configured to emit ultrasound energy, a controller (108) operably coupled to the transducer (104), the controller (108) being configured to operate the transducer (104) in an imaging mode wherein images of the tissue, blood flow information, and three-dimensional volumetric data are obtained and in a therapy mode wherein portions of the tissue in the tissue treatment area (10) are treated with a high - intensity focused ultrasound (HIFU) therapy by the transducer (104), the controller (108) being further configured to automatically generate a proposed treatment plan for providing HIFU therapy to the tissue treatment area (10), the proposed treatment plan including selection of a plurality of treatment sites to receive HIFU therapy, the plurality of treatment sites of the proposed treatment plan being selected to provide HIFU therapy to a first tissue component, which excluding a second tissue component from HIFU therapy, **characterized in that** the transducer (104) is configured to sense ultrasound energy, the images being obtained in the imaging mode by ultrasound energy sensed by the transducer; **in that** the controller (108) is further configured to generate the proposed treatment plan by generation of a three-dimensional model of the tissue treatment area based on the ultrasound data obtained from the transducer in the imaging mode, the treatment sites being selected in the tissue treatment area (10), and on the selection of a plurality of lesions from a lesion library (113), wherein the parameters of a lesion in the lesion library includes lesion size and ultrasound transducer configuration, wherein the selection of a plurality of lesions includes a first gross filling of the tissue treatment area (10) with proposed lesions and a second fine filling of the tissue treatment area (10), and wherein open regions of the area (10) are filled with smaller proposed lesions from the lesion library (113).

2. The apparatus of claim 1, wherein the tissue treatment area (10) corresponds to a prostate (11) of a patient and wherein the transducer is contained within a probe (102), the probe (102) being configured for transrectal insertion to position the transducer proximate to the tissue treatment area (10).

3. The apparatus of claim 1, wherein the first tissue component corresponds to a prostatic capsule (12), and the second tissue component corresponds to a neuro-vascular bundles (20).

4. The apparatus of any preceding claim, wherein the location of the first tissue component is determined based on a three-dimensional model of the first tissue component, the three-dimensional model being generated by the steps of:

   locating a first boundary trace of the first tissue component in a first image obtained during the imaging mode of operation, the first image corresponding to a first plane;
   locating a second boundary trace of the first tissue component in a second image obtained during the imaging mode of operation, the second image corresponding to a second plane, the second plane being orthogonal to the first plane;
   computing a boundary surface of the tissue component based on the first boundary trace and the second boundary trace.

5. The apparatus of any preceding claim, further comprising a display device (112), the controller (108) being configured to present with the display device (112) for review by a user a three-dimensional representation of the tissue treatment area (10) a plurality of treatment, indicia, each of the treatment indicia corresponding to a respective treatment site in the proposed treatment plan, a representation of a boundary of the first tissue component, the boundary being determined from a three-dimensional model of the first tissue component; and a blood flow indicia indicating the location of the second tissue component.

6. The apparatus of claim 5, wherein the blood flow indicia provides an indication of the amount of blood flow.

7. The apparatus of either claim 5 or claim 6 further comprising a user input device (110), the controller (108) being configured to generate a modified proposed treatment plan based on a requested modification received with the user input device (110).

8. The apparatus of any preceding claim, wherein the controller (108) includes a multiple gate system for sampling Doppler information which provides the blood flow information, each gate (212, 242) of the multiple gate system being configured to sample a given time period of Doppler information.

9. The apparatus of any preceding claim, wherein the exclusion of the tissue component is based on the detection of blood flow at a location corresponding to the tissue component.

10. The apparatus of claim 9, wherein the exclusion is further based on the location of the tissue component relative to the three-dimensional model of the first tissue component.

11. The apparatus of any one of claims 1 to 8, wherein the controller (108) excludes a tissue component from the proposed treatment plan based on the location of the tissue component.

12. The apparatus of claim 11, wherein the location of the excluded tissue component is based on a three-dimensional model of the tissue component, the three-dimensional model of the tissue component being based on the generated ultrasound data.

13. The apparatus of any preceding claim, wherein the controller (108) is configured to commence the treatment plan such that substantially all of an interior of a selected tissue component in the tissue treatment area (10) is treated with HIFU therapy in the proposed treatment plan.

14. The apparatus of any preceding claim, wherein the imaging by the transducer (104) includes multiple two-dimensional images of the tissue treatment area, wherein the imaging is a plurality of sector images and a plurality of linear images.

15. The apparatus of any one of claims 1 to 14, wherein the three-dimensional volumetric data includes three-dimensional imaging data.

**Patentansprüche**

1. Vorrichtung zum Behandeln eines Gewebebehandlungsbereichs mit mehreren Gewebekomponenten, wobei die Vorrichtung Folgendes umfasst: einen Wandler (104), der in Bezug auf den Gewebebehandlungsbereich (10) positioniert werden kann, wobei der Wandler (104) zum Emittieren von Ultraschallenergie konfiguriert ist, eine Steuerung (108), die mit dem Wandler (104) operativ gekoppelt ist, wobei die Steuerung (108) zum Betreiben des Wandlers (104) in einem Abbildungsmodus, in dem Bilder des Gewebes, Blutflussinformationen sowie dreidimensionale volumetrische Daten erhalten werden, und in einem Therapiemodus konfiguriert ist, in dem Teile des Gewebes in dem Gewebebehandlungsbereich (10) von dem Wandler (104) mit einer HIFU-(hochintensiver fokussierter Ultraschall)-Therapie behandelt werden, wobei die Steuerung (108) ferner zum automatischen Erzeugen eines vorgeschlagenen Behandlungsplans zum Durchführen von HIFU-Therapie für den Gewebebehandlungsbereich (10) konfiguriert ist, wobei der vorgeschlagene Behandlungsplan das Auswählen von mehreren Behandlungsstellen zum Durchführen von HIFU-Therapie beinhaltet, wobei die mehreren Behandlungsstellen des vorgeschlagenen Behandlungsplans zum Durchführen von HIFU-Therapie an einer ersten Gewebekomponente ausgewählt werden, während eine zweite Gewebekomponente von der HIFU-Therapie ausgeschlossen wird, **dadurch gekennzeichnet, dass** der Wandler (104) zum Erfassen von Ultraschallenergie konfiguriert ist, wobei die Bilder im Abbildungsmodus durch vom Wandler erfasste Ultraschallenergie erhalten werden; dadurch, dass die Steuerung (108) ferner konfiguriert ist zum Erzeugen des vorgeschlagenen Behandlungsplans durch Erzeugen eines dreidimensionalen Modells des Gewebebehandlungsbereichs auf der Basis der vom Wandler im Abbildungsmodus erhaltenen Ultraschalldaten, wobei die Behandlungsstellen im Gewebebehandlungsbereich (10) ausgewählt werden, und der Auswahl von mehreren Läsionen aus einer Läsionsbibliothek (113), wobei die Parameter einer Läsion in der Läsionsbibliothek die Läsionsgröße und die Ultraschallwandlerkonfiguration beinhalten, wobei die Auswahl von mehreren Läsionen eine erste Grobfüllung des Gewebebehandlungsbereichs (10) mit vorgeschlagenen Läsionen und eine zweite Feinfüllung des Gewebebehandlungsbereichs (10) beinhaltet, und wobei offene Regionen des Bereichs (10) mit kleineren vorgeschlagenen Läsionen aus der Läsionsbibliothek (113) gefüllt werden.

2. Vorrichtung nach Anspruch 1, wobei der Gewebebehandlungsbereich (10) einer Prostata (11) eines Patienten entspricht und wobei der Wandler in einer Sonde (102) enthalten ist, wobei die Sonde (102) für eine transrektale Einführung zu der Position des Wandlers in der Nähe des Gewebebehandlungsbereichs (10) konfiguriert ist.

3. Vorrichtung nach Anspruch 1, wobei die erste Gewebekomponente einer Prostatakapsel (12) entspricht und die zweite Gewebekomponente Nervengefäßbündeln (20) entspricht.

4. Vorrichtung nach einem vorherigen Anspruch, wobei der Ort der ersten Gewebekomponente auf der Basis eines dreidimensionalen Modells der ersten Gewebekomponente ermittelt wird, wobei das dreidimensionale Modell mit den folgenden Schritten erzeugt wird:

Finden einer ersten Begrenzungsspur der ersten Gewebekomponente in einem ersten Bild, das im Abbildungsbetriebsmodus erhalten wurde, wobei das erste Bild einer ersten Ebene entspricht;
Finden einer zweiten Begrenzungsspur der ersten Gewebekomponente in einem zweiten Bild, das im Abbildungsbetriebsmodus erhalten wurde, wobei das zweite Bild einer zweiten Ebene entspricht, wobei die zweite Ebene orthogonal zur ersten Ebene ist;
Berechnen einer Begrenzungsfläche der Gewebekomponente auf der Basis der ersten Begrenzungsspur und der zweiten Begrenzungsspur.

5. Vorrichtung nach einem vorherigen Anspruch, die ferner eine Anzeigevorrichtung (112) umfasst, wobei die Steuerung (108) so konfiguriert ist, dass sie einem Benutzer über die Anzeigevorrichtung (112) Folgendes zur Ansicht präsentiert: eine dreidimensionale Darstellung des Gewebebehandlungsbereichs (10), mehrere Behandlungsindizien, wobei jedes der Behandlungsindizien einer jeweiligen Behandlungsstelle in dem vorgeschlagenen Behandlungsplan entspricht, eine Darstellung einer Begrenzung der ersten Gewebekomponente, wobei die Begrenzung anhand eines dreidimensionalen Modells der ersten Gewebekomponente bestimmt wird; und ein Blutflussindizium, das den Ort der zweiten Gewebekomponente anzeigt.

6. Vorrichtung nach Anspruch 5, wobei das Blutstromindizium eine Anzeige für die Blutflussmenge gibt.

7. Vorrichtung nach Anspruch 5 oder Anspruch 6, die ferner eine Benutzereingabevorrichtung (110) umfasst, wobei die Steuerung (108) zum Erzeugen eines modifizierten vorgeschlagenen Behandlungsplans auf der Basis einer mit der Benutzereingabevorrichtung (110) empfangenen angeforderten Modifikation konfiguriert ist.

8. Vorrichtung nach einem vorherigen Anspruch, wobei die Steuerung (108) ein Mehrgattersystem zum Abtasten von Doppler-Informationen beinhaltet, die die Blutflussinformationen geben, wobei jedes Gatter (212, 242) des Mehrgattersystems zum Abtasten einer gegebenen Zeitperiode von Doppler-Informationen konfiguriert ist.

9. Vorrichtung nach einem vorherigen Anspruch, wobei der Ausschluss der Gewebekomponente auf der Erkennung von Blutfluss an einem Ort basiert, der der Gewebekomponente entspricht.

10. Vorrichtung nach Anspruch 9, wobei der Ausschluss ferner auf dem Ort der Gewebekomponente relativ zu dem dreidimensionalen Modell der ersten Gewebekomponente basiert.

11. Vorrichtung nach einem der Ansprüche 1 bis 8, wobei die Steuerung (108) eine Gewebekomponente von dem vorgeschlagenen Behandlungsplan auf der Basis des Ortes der Gewebekomponente ausschließt.

12. Vorrichtung nach Anspruch 11, wobei der Ort der ausgeschlossenen Gewebekomponente auf einem dreidimensionalen Modell der Gewebekomponente basiert, wobei das dreidimensionale Modell der Gewebekomponente auf den erzeugten Ultraschalldaten basiert.

13. Vorrichtung nach einem vorherigen Anspruch, wobei die Steuerung zum Beginnen des Behandlungsplans konfiguriert ist, so dass im Wesentlichen das gesamte Innere einer gewählten Gewebekomponente in dem Gewebebehandlungsplan (10) mit HIFU-Therapie in dem vorgeschlagenen Behandlungsplan behandelt wird.

14. Vorrichtung nach einem vorherigen Anspruch, wobei die Abbildung durch den Wandler (104) mehrere zweidimensionale Bilder des Gewebebehandlungsbereichs beinhaltet, wobei die Abbildung eine Mehrzahl von Sektorbildern und eine Mehrzahl von linearen Bildern ist.

15. Vorrichtung nach einem der Ansprüche 1 bis 14, wobei die dreidimensionalen volumetrischen Daten dreidimensionale Abbildungsdaten beinhalten.

**Revendications**

1. Appareil pour le traitement d'une zone de traitement tissulaire comprenant une pluralité de composants tissulaires, l'appareil comprenant un transducteur (104) qui est positionnable par rapport à la zone de traitement tissulaire (10), le transducteur (104) étant configuré pour émettre une énergie à ultrasons, un contrôleur (108) couplé d'une manière opérationnelle au transducteur (104), le contrôleur (108) étant configuré pour faire fonctionner le transducteur (104) dans un mode d'imagerie dans lequel des images du tissu, des informations de flux sanguin et des données volumétriques tridimensionnelles sont obtenues, et dans un mode de thérapie dans lequel des parties du tissu dans la zone de traitement tissulaire (10) sont traitées par thérapie par ultrasons focalisés de haute intensité (HIFU) par le transducteur (104), le contrôleur (108) étant configuré en outre pour générer automatiquement un plan de traitement proposé pour fournir la thérapie HIFU à la zone de traitement tissulaire (10), le plan de traitement proposé comprenant une sélection d'une pluralité de sites de traitement pour recevoir la thérapie HIFU, la pluralité de sites de traitement du plan de traitement proposé étant sélectionnée dans le but de fournir la thérapie HIFU à un premier composant tissulaire, tout en excluant un deuxième composant tissulaire de la thérapie HIFU; **caractérisé en ce que** le transducteur (104) est configuré pour détecter l'énergie à ultrasons, les images étant obtenues dans le mode d'imagerie par l'énergie à ultrasons détectée par le transducteur; **en ce que** le contrôleur (108) est configuré en outre pour générer le plan de traitement proposé en générant un modèle tridimensionnel de la zone de traitement tissulaire basé sur les données des ultrasons obtenues du transducteur dans le mode d'imagerie, les sites de traitement étant sélectionnés dans la zone de traitement tissulaire (10), et sur la sélection d'une pluralité de lésions d'une banque de lésions (113), où les paramètres d'une lésion dans la banque de lésions comprennent la taille de la lésion et la configuration du transducteur à ultrasons, où la sélection d'une pluralité de lésions comprend un premier remplissage grossier de la zone de traitement tissulaire (10) avec les lésions proposées et une deuxième remplissage fin de la zone de traitement tissulaire (10) et où les régions ouvertes de la zone (10) sont remplies avec de plus petites lésions proposées de la banque de lésions (113).

2. Appareil selon la revendication 1, dans lequel la zone de traitement tissulaire (10) correspond à une prostate (11) d'un patient et dans lequel le transducteur est contenu dans une sonde (102), la sonde (102) étant configurée pour insertion transrectale afin de positionner le transducteur à côté de la zone de traitement tissulaire (10).

3. Appareil selon la revendication 1, dans lequel le premier composant tissulaire correspond à une capsule prostatique (12) et le deuxième composant tissulaire correspond à des faisceaux neurovasculaires (20).

4. Appareil selon l'une quelconque des revendications précédentes, dans lequel l'emplacement du premier composant tissulaire est déterminé sur la base d'un modèle tridimensionnel du premier composant tissulaire, le modèle tridimensionnel étant généré par les étapes consistant à :

   localiser une première trace limite du premier composant tissulaire dans une première image obtenue pendant le fonctionnement en mode imagerie, la première image correspondant à un premier plan;
   localiser une deuxième trace limite du premier composant tissulaire dans une deuxième image obtenue pendant le fonctionnement en mode d'imagerie, la deuxième image correspondant à un deuxième plan, le deuxième plan étant généralement orthogonal au premier plan;
   calculer une surface limite du composant tissulaire sur la base de la première trace limite et de la deuxième trace limite.

5. Appareil selon l'une quelconque des revendications précédentes, comprenant en outre un dispositif d'affichage (112), le contrôleur (108) étant configuré pour présenter à l'aide du dispositif d'affichage (112) pour examen par un utilisateur, une représentation tridimensionnelle de la zone de traitement tissulaire (10), une pluralité d'indices de traitement, chacun des indices de traitement correspondant à un site de traitement respectif dans le plan de traitement proposé, une représentation d'une limite du premier composant tissulaire, la limite étant déterminée à partir d'un modèle tridimensionnel du premier composant tissulaire; et un indice de flux sanguin indiquant l'emplacement du deuxième composant tissulaire.

6. Appareil selon la revendication 5, dans lequel l'indice de flux sanguin fournit une indication du volume de flux sanguin.

7. Appareil selon la revendication 5 ou la revendication 6, comprenant en outre un dispositif d'entrée utilisateur (110), le contrôleur (108) étant configuré pour générer un plan de traitement proposé modifié sur la base d'une modification requise reçue avec le dispositif d'entrée utilisateur (110).

8. Appareil selon l'une quelconque des revendications précédentes, dans lequel le contrôleur (108) comprend un système multiporte pour échantillonner des informations Doppler qui fournissent les informations de flux sanguin, chaque porte (212, 242) du système multiporte étant configurée pour échantillonner une période de temps donnée des informations Doppler.

9. Appareil selon l'une quelconque des revendications précédentes, dans lequel l'exclusion du composant tissulaire est basée sur la détection d'un flux sanguin à un emplacement correspondant au composant tissulaire.

10. Appareil selon la revendication 9, dans lequel l'exclusion est basée en outre sur l'emplacement du composant tissulaire par rapport au modèle tridimensionnel du premier composant tissulaire.

**11.** Appareil selon l'une quelconque des revendications 1 à 8, dans lequel le contrôleur (108) exclut un composant tissulaire du plan de traitement proposé sur la base de l'emplacement du composant tissulaire.

**12.** Appareil selon la revendication 11, dans lequel l'emplacement du composant tissulaire exclu est basé sur un modèle tridimensionnel du composant tissulaire, le modèle tridimensionnel du composant tissulaire étant basé sur les données générées des ultrasons.

**13.** Appareil selon l'une quelconque des revendications précédentes, dans lequel le contrôleur (108) est configuré pour commencer le plan de traitement de telle sorte que sensiblement tout l'intérieur d'un composant tissulaire sélectionné dans la zone de traitement tissulaire (10) est traité par thérapie HIFU dans le plan de traitement proposé.

**14.** Appareil selon l'une quelconque des revendications précédentes, dans lequel l'imagerie par le transducteur (104) comprend de multiples images bidimensionnelles de la zone de traitement tissulaire, où l'imagerie est une pluralité d'images sectorielles et une pluralité d'images linéaires.

**15.** Appareil selon l'une quelconque des revendications 1 à 14, dans lequel les données volumétriques tridimensionnelles comprennent des données d'imagerie tridimensionnelle.

*Fig. 1*

100

TISSUE TREATMENT AREA

16 SEMINAL VESICLE

EJACULATORY DUCT

BLADDER

10

11

18 RECTAL WALL

PROSTATIC CAPSULE

17

20

NERVE BUNDLES

URETHRA

12

14

112 DISPLAY

106 POSITIONING MEMBER

104 TRANSDUCER PROBE

115

116

114

110 USER INPUT DEVICE

108 CONTROLLER

105

102

111 SOFTWARE 109 MEMORY

LESION LIBRARY

113

EP 1 755 458 B1

*Fig. 2*

*Fig. 2A*

*Fig. 3*

EP 1 755 458 B1

Fig. 4A

*Fig. 4B*

*Fig. 4C*

EP 1 755 458 B1

300

DETERMINE THE LOCATION OF TISSUE COMPONENTS WITHIN THE TISSUE TREATMENT AREA WITH TRANSDUCER MEMBER ⟶302

DETERMINE THE LOCATION OF BLOOD FLOW WITHIN THE TISSUE TREATMENT AREA WITH DOPPLER IMAGING TECHNIQUES ⟶304

DISPLAY REPRESENTATION OF TISSUE TREATMENT AREA INCLUDING A REPRESENTATION OF THE LOCATIONS WITHIN THE TISSUE TREATMENT AREA CORRESPONDING TO BLOOD FLOW ⟶306

DISPLAY REPRESENTATIONS OF SUGGESTED TREATMENT ZONES BASED ON THE IDENTIFIED LOCATIONS OF THE VARIOUS TISSUE COMPONENTS AND BLOOD FLOW ⟶308

RECEIVE INPUT ON TREATMENT ZONES TO ADD OR EXCLUDE FROM TREATMENT ⟶310

INITIATE THERAPY TO TISSUE CORRESPONDING TO THE NON-EXCLUDED TREATMENT ZONES ⟶312

*FIG. 5*

400

402

BEGIN → PATIENT AND SYSTEM PREP

404

412

410

DETECT NVB'S USING DOPPLER

ACQUIRE 3D ULTRASOUND IMAGES OF PROSTATE

406

LESION LIBRARY (113) AND TREATMENT PARAMETERS

(454, 456, 458)

TRACE AND IDENTIFY STRUCTURES

408

414

MODEL STRUCTURES COMPUTE SURFACES

416

AUTOMATIC TREATMENT PLANNING MODULE

425

423

422

MANUAL TREATMENT PLAN → DISPLAY/REVIEW TREATMENT PLAN

427

424

TREAT AND MONITOR TREATMENT → END

*Fig. 6A*

INPUTS

SHAPE MODELS — 450

NVB LOCATION — 452

TRANSDUCER PARAMETERS: — 454
- FOCAL LENGTH
- LESION SIZE FOR GIVEN POWER LEVEL (FROM IN-VIVO DATA OR LESION LIBRARY)

INCLUSION/EXCLUSION — 456

TREATMENT PARAMETERS:
- MARGIN
- WHOLE VS. PARTIAL ABLATION
- LESION OVERLAP, ETC. — 458

416

ALGORITHM:

POPULATE PROSTATE VOLUME WITH ELEMENTARY LESIONS, FROM ANTERIOR TO POSTERIOR LOCATIONS, UNTIL THE ENTIRE VOLUME IS POPULATED, BASED ON THE GEOMETRICAL, SYSTEM (TRANSDUCER AND PROBE), AND PHYSICIAN CONSTRAINTS TO MINIMIZE THE NUMBER OF LESIONS, PHYSICAL PROBE ROTATION, OR FOCAL LENGTH CHANGE.

ALLOW FOR THE ADDITION/REMOVAL OF EXCLUSION ZONES, AND INTERACTIVE TREATMENT PLAN REVIEW.

GENERATE TREATMENT PLAN.

DISPLAY/REVIEW TREATMENT PLAN — 422

TREAT AND MONITOR TREATMENT — 424

OUTPUTS

*Fig. 6B*

480

```
┌─────────────────────────┐
│   POPULATE THE PROSTATE │
│   VOLUME DEFINED BY THE  │
│   SHAPE MODEL OF THE     │──482
│   PROSTATIC CAPSULE AND  │
│   OTHER PARAMETERS SUCH  │
│        AS MARGIN         │
└─────────────────────────┘
            │
            ▼
┌─────────────────────────┐
│   STORE PARAMETERS FOR   │
│   EACH TREATMETN SITE    │
│   SUCH AS TRANSDUCER     │──484
│      FOCAL LENGTH        │
└─────────────────────────┘
            │
            ▼
┌─────────────────────────┐
│  IDENTIFY TREATMENT SITES│
│   TO BE EXCLUDED FROM    │──486
│       TREATMENT          │
└─────────────────────────┘
            │
            ▼
┌─────────────────────────┐
│   DETERMINE ORDER OF     │
│  TREATMENT FOR PROPOSED  │──488
│    TREATMENT SITES       │
└─────────────────────────┘
```

*Fig. 7*

508

Trace Mode | Trace | Erase | Show | Refine | Delete | Point Mode | Click | Delete | Print

514

500A

516g
516h

516i
516j
516k

516f
516e
512
516d
516c
516b
516a

516m    516n

510G
510H
510I

Hide

Capsule | Rectal Wall | Seminal 1 | Seminal 2 | Urethra | NVB | EXCLUDE

510a    510b    510c    510d    510e    510f    518

*Fig. 8A*

*Fig. 8B*

EP 1 755 458 B1

*Fig. 9*

*Fig. 10A*

*Fig. 10B*

112

SECTOR IMAGE
600A

SECTOR IMAGE
600B

SECTOR IMAGE
600C

SECTOR IMAGE
600D

SECTOR IMAGE
600E

SECTOR IMAGE
600F

SECTOR IMAGE
600G

SECTOR IMAGE
600H

SECTOR IMAGE
600I

*Fig. 11*

Fat Layer

Prostate

Treatment Zones

Rectal Wall

624

*Fig. 12*

624    702          702    532
       534

*Fig. 13*

113

| | 802 | 804 | 806 |
|---|---|---|---|
| | Lesion Size | Parameters | Available |
| 800A | Size A | Parameter A | YES |
| 800B | Size B | Parameter B | YES |
| 800C | Size C | Parameter C | NO |

800

*Fig. 14*

800D 800E 800F 800G 800H 800I 800J 800K

10W 12W 14W 16W 18W 20W 22W 24W

810

800L 800M 800N 800O 800P 800Q 800R 800S

26W 28W 30W 32W 34W 36W 38W 40W

*Fig. 15*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5762066 A
- US 84050292 A
- AU 5732801
- CA 1332441
- CA 2250081
- US 5036855 A
- US 5117832 A
- US 5492126 A
- US 6685640 B
- WO 0157777 A
- US 6618620 B
- US 07037105 A
- US 56855604 P